(19) Europäisches Patentamt / European Patent Office / Office européen des brevets

(11) **EP 4 574 039 A1**

(12) **EUROPEAN PATENT APPLICATION**

(43) Date of publication:
25.06.2025 Bulletin 2025/26

(21) Application number: 24221988.9

(22) Date of filing: 20.12.2024

(51) International Patent Classification (IPC):
*A61B 5/1495* (2006.01)

(52) Cooperative Patent Classification (CPC):
**A61B 5/7203; A61B 5/14532; A61B 5/1495;**
A61B 2560/0223

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: 22.12.2023  KR 20230190089
05.11.2024  KR 20240155176

(71) Applicant: **i-Sens, Inc.**
**Seoul 06646 (KR)**

(72) Inventor: **LEE, Da Vid**
**06646 Seoul (KR)**

(74) Representative: **Isarpatent**
**Patent- und Rechtsanwälte**
**Barth Hassa Peckmann & Partner mbB**
**Friedrichstraße 31**
**80801 München (DE)**

(54) **APPARATUS FOR MONITORING GLUCOSE AND METHOD THEREOF**

(57)    An apparatus for monitoring glucose according to an embodiment of the present invention includes an input unit that obtains first biometric information including a plurality of data points measuring glucose-related information of a subject; a noise removal unit that removes noise information included in the first biometric information; a preprocessing unit that preprocesses the first biometric information from which the noise information is removed to generate second biometric information having a lower sampling rate than the first biometric information; a compensation unit that generates compensation data based on the second biometric information and generates a calibration algorithm based on the compensation data; and a glucose level acquisition unit that acquires a glucose level related to the second biometric information by reflecting the calibration algorithm and a set time delay.

FIG. 1

**Description**

BACKGROUND OF THE INVENTION

Field of the Invention

Embodiments relate to an apparatus for monitoring glucose capable of continuously monitoring and providing user's glucose, and a method thereof.

Description of the Related Art

Recently, with the advancement of medical technology, various medical devices that are attached to a user's body have been developed and sold. The medical devices that are attached to the user's body may be usefully used to monitor biometric information or provide treatment by being attached to skins of patients with chronic diseases.

For example, chronic diseases such as diabetes need to be continuously managed. To monitor glucose of a diabetic patient, a medical device that is attached to the skin to measure the glucose may be used. Diabetes is characterized by almost no symptoms in the early stages. As the disease progresses, symptoms specific to diabetes such as overdrinking, polyphagia, polyuria, weight loss, general ennui, skin itching, and longlasting wounds of hands and feet appear. As the diabetes progresses further, complications such as visual impairment, hypertension, kidney disease, stroke, periodontal disease, muscle cramps and neuralgia, and gangrene appear. In order to diagnose such diabetes and prevent the diabetes from progressing to complications, systematic glucose measurement and treatment needs to be performed in parallel.

For diabetics and people who have not progressed to diabetes but have more sugar detected in their blood than normal, many medical device manufacturers provide various types of blood glucose meters that may measure glucose.

For the blood glucose meters, there may be a method in which a user measures glucose once by drawing blood from his/her fingertip and a method in which users continuously measure glucose by attaching an apparatus for monitoring glucose to their abdomen, arm, etc.

Diabetics generally experience high and low glucose states, but emergencies occur when the diabetics are in a low glucose state, and diabetics may lose consciousness or even die when the diabetics remain in the low glucose state for a long time without sugar intake. Therefore, immediate detection of the low glucose state is very important for diabetic patients. However, there is a limit to accurately detecting the low glucose state with a blood-collection type blood glucose meter that measures glucose intermittently.

Recently, to overcome the limitation, a continuous glucose monitoring system (CGMS) that is inserted into a body to measure glucose levels at intervals of several minutes has been developed and used. The CGMS may continuously measure glucose by inserting a needle-shaped transdermal sensor into relatively less painful areas such as an abdomen and an arm to minimize users' pain and discomfort due to blood collection.

The CGMS is configured to include a sensor transmitter that is inserted into a user's skin to measure glucose in the body and transmit the measured glucose level, a terminal that outputs the obtained glucose level, etc.

Since the glucose is measured while the sensor transmitter is attached to the body and a part of the sensor is inserted into the body, the sensor inserted into the body may be recognized as foreign matters. That is, a biological reaction - for example, a hydration reaction - to a sensor may occur. This may cause a change in sensitivity (slope) at the initial stage of sensor insertion. This may decrease the accuracy of the sensor and make it difficult to maintain the service life. The changed slope may decrease the accuracy of the CGMS by acquiring an excessive or insufficient glucose level for the measured biosignal. In the past, in order to compensate for the change in sensitivity (slope), a method in which a simple sensitivity obtained by dividing a reference glucose level input by a user - a glucose level measured by a self-blood glucose monitoring system (BGMS) - by a current value of a sensor and a preset offset value are used or a method in which the sensitivity is determined through regression analysis on a reference glucose level and the corresponding current value has been developed. However, there may be a problem in that the method does not respond sensitively to abnormal glucose levels or abnormal operation of the sensor, and thus provides inaccurate sensitivity and offset values.

SUMMARY OF THE INVENTION

An embodiment may provide an apparatus for monitoring glucose capable of continuously monitoring user's glucose and providing the monitored user's glucose to a user, and a method thereof.

An embodiment may provide an apparatus for monitoring glucose capable of providing accurate glucose information by removing noise from a signal, and a method thereof.

An embodiment may provide an apparatus for monitoring glucose capable of providing accurate glucose information by applying accurate sensor sensitivity, and a method thereof.

An embodiment may provide an apparatus for monitoring glucose capable of providing accurate glucose information through a glucose acquiring process that is tailored to various environments, and a method thereof.

The problem to be solved in the embodiment is not limited thereto, and the purpose or effect that may be grasped from the means or embodiment of the problem to be described below is included.

According to an embodiment of the present invention, an apparatus for monitoring glucose may include: an input unit that obtains first biometric information including a plurality of data points measuring glucose-related information of a subject; a noise removal unit that removes noise information included in the first biometric information; a preprocessing unit that preprocesses the first biometric information from which the noise information is removed to generate second biometric information having a lower sampling rate than the first biometric information; a compensation unit that generates compensation data based on the second biometric information and generates a calibration algorithm based on the compensation data; and a glucose level acquisition unit that acquires a glucose level related to the second biometric information by reflecting the calibration algorithm and a set time delay.

The noise removal unit may perform a first noise removal process of removing a data point determined as noise information among first data points based on a plurality of data points included in a set time interval, and perform a second noise removal process of adjusting a value of the first data point based on a plurality of data points included in a set time interval to remove set noise information.

The noise removal unit may perform the first noise removal process of determining whether any one of the data points is an outlier using a data point other than any one of the plurality of data points included in the set time interval, and removing any one of the data points based on being determined that any one of the data points is the outlier.

The noise removal unit may perform the first noise removal process of acquiring a reference value including at least one of an average slope, a slope change value, an average value, and a standard deviation using the data point other than any one of the plurality of data points, and comparing the reference value with the one data point to determine whether the one data point is the outlier.

The noise removal unit may perform the second noise removal process of acquiring a weight for any one of the data points using a data point other than any one of the plurality of data points, and applying the weight to the one data point to remove a noise component included in the one data point.

The preprocessing unit may perform a first preprocessing process of preprocessing the first biometric information from which the noise information is removed based on the first biometric information from which the noise information is removed and first time interval.

The preprocessing unit may perform the first preprocessing process of dividing a plurality of data points included in the first biometric information from which the noise information is removed at the first time interval, removing a first data point related to a set upper value and a second data point related to a set lower value among a plurality of data points at each first time interval, and acquiring an average value of the plurality of data points from which the first data point and the second data point are removed at each first time interval as the first preprocessing data.

The preprocessing unit may perform a second preprocessing process of preprocessing the first preprocessing data based on the first preprocessing data and second time interval having a value greater than the first time interval.

The preprocessing unit may perform the second preprocessing process of dividing a plurality of average values included in the first preprocessing data at the second time interval, removing a first average value related to a set upper value and a second average value related to a set lower value among the plurality of average values at each second time interval, acquiring an average value for the plurality of average values from which the first average value and the second average value are removed at each second time interval as second preprocessing data, and determining the second preprocessing data as the second biometric information.

The compensation unit may determine any one of values of pre-stored table data as an offset value based on the second biometric information, and apply the offset value to the second biometric information to generate the compensation data.

The compensation unit may acquire an average value of data points for a set period included in the second biometric information, apply the average value to the second biometric information to generate ideal data having a set baseline, generate a first weight and a second weight, and add up a value obtained by applying the first weight to the second biometric information and a value obtained by applying the second weight to the ideal data to generate the compensation data.

The first weight and the second weight may have a negative correlation.

The compensation unit may perform at least one of: a first sensor sensitivity determination process of determining a first sensitivity based on the compensation data and a set reference glucose level, a second sensor sensitivity determination process of determining a second sensitivity based on a sensor sensitivity set in response to a glucose measurement sensor and the first sensitivity, a third sensor sensitivity determination process of adjusting the second sensitivity based on a first previous time point sensitivity and a first previous time point final sensitivity that are determined at a previous time point, and determining the adjusted second sensitivity as the third sensitivity, and a fourth sensor sensitivity determination process of adjusting the third sensitivity according to whether a glucose level acquired based on the third sensitivity falls within a set range, and determining the adjusted third sensitivity as a fourth sensitivity, and may determine one of the first sensitivity, the second sensitivity, the third sensitivity, and the fourth sensitivity as the final sensitivity.

The compensation unit may perform the second sensor sensitivity determination process of determining the sensor sensitivity as the second sensitivity based on that the first sensitivity is higher than or equal to the sensor sensitivity, and determining the processed sensor sensitivity, which is obtained by processing the sensor sensitivity through a set algorithm, as the second sensitivity based on that the first sensitivity is lower than the sensor sensitivity.

The compensation unit may perform the third sensor sensitivity determination process of determining an average value of the first sensitivity and the second sensitivity as the third sensitivity based on that a state in which the first sensitivity determined at a previous time point is higher or lower than the final sensitivity at the corresponding previous time point occurs continuously.

The compensation unit may perform the fourth sensor sensitivity determination process of acquiring a glucose level related to the compensation data using the third sensitivity, determining the third sensitivity as the fourth sensitivity when the glucose level falls within a preset range, and determining the fourth sensitivity based on a glucose level at the previous time point, a reference glucose level, and the compensation data when the glucose level does not fall within the preset range.

The glucose level acquisition unit may compensate for the time delay in the second biometric information to generate time-compensated biometric information, and apply the time-compensated biometric information to the calibration algorithm to acquire the glucose level.

According to another embodiment of the present invention, a method of monitoring glucose using an apparatus for monitoring glucose may include: obtaining, by the apparatus for monitoring glucose, first biometric information including a plurality of data points measuring glucose-related information of a subject; removing, by the apparatus for monitoring glucose, noise information included in the first biometric information; preprocessing, by the apparatus for monitoring glucose, the first biometric information from which the noise information is removed to generate second biometric information having a lower sampling rate than the first biometric information; generating, by the apparatus for monitoring glucose, compensation data based on the second biometric information; and generating a calibration algorithm based on the compensation data; and acquiring, by the apparatus for monitoring glucose, a glucose level related to the second biometric information by reflecting the calibration algorithm and a set time delay.

According to the embodiment, it is possible to provide the accurate glucose information in various environments.

Various and beneficial advantages and effects of the present inventive concept are not limited to the above description, and may be more easily understood in the course of describing the specific embodiments of the present inventive concept.

BRIEF DESCRIPTION OF THE DRAWINGS

FIG. 1 is a diagram for describing a system for monitoring glucose according to an embodiment of the present invention.
FIG. 2 is a configuration diagram of an apparatus for monitoring glucose according to an embodiment of the present invention.
FIG. 3 is a flowchart of a method of monitoring glucose according to an embodiment of the present invention.
FIG. 4 is a flowchart illustrating in detail step S320 of FIG. 3.
FIG. 5 is a diagram for describing an outlier filtering process according to an embodiment of the present invention.
FIG. 6 is a diagram for describing a low pass filtering process according to an embodiment of the present invention.
FIG. 7 is a flowchart illustrating in detail step S330 of FIG. 3.
FIG. 8 is a diagram for describing a process of generating a first preprocessing process according to an embodiment of the present invention.
FIG. 9 is a diagram for describing a process of generating a second preprocessing process according to an embodiment of the present invention.
FIG. 10 is an exemplary diagram of an electrical signal measured by a sensor according to an embodiment.
FIG. 11 is an exemplary diagram of an electrical signal showing a trend according to an embodiment.
FIG. 12 is an exemplary diagram of a biosignal showing a syllable fragmentation phenomenon according to an embodiment.
FIG. 13 is a flowchart of an embodiment illustrating in detail step S340 of FIG. 3.
FIG. 14 is a flowchart of an embodiment illustrating in detail step S340 of FIG. 3.
FIG. 15 is an exemplary diagram of an ideal signal from which the trend is removed and to which an average value is applied according to an embodiment of the present invention.
FIG. 16 is a diagram for describing a process of generating compensation data, which is second biometric information compensated from second biometric information and ideal data, according to an embodiment of the present invention.
FIG. 17 is a diagram for describing a weight applied to the second biometric information and the ideal data according to an embodiment.
FIG. 18 is a flowchart of another embodiment illustrating in detail step S350 of FIG. 3.
FIG. 19 is a diagram for describing a process of determining a second sensitivity according to an embodiment of the present invention.

FIG. 20 is a diagram for describing a process of determining a third sensitivity according to an embodiment of the present invention.

FIG. 21 is a diagram for describing an embodiment that determines a fourth sensitivity according to an embodiment of the present invention.

FIG. 22 is a diagram for describing another embodiment that determines the fourth sensitivity according to an embodiment of the present invention.

FIG. 23 is a diagram for describing another embodiment that determines the fourth sensitivity according to an embodiment of the present invention.

FIG. 24 is a flowchart illustrating a method of acquiring sensitivity according to another embodiment.

FIG. 25 is a flowchart illustrating a method of adjusting sensitivity according to a period that has elapsed since a sensor illustrated in FIG. 24 is inserted into a body.

FIG. 26 is an exemplary diagram illustrating adjusting sensitivity according to a period that has elapsed since the sensor illustrated in FIG. 25 is inserted into the body.

FIG. 27 is a flowchart illustrating a method of adjusting sensitivity based on an average sensing value and an average glucose level illustrated in FIG. 24.

FIG. 28 is an exemplary diagram illustrating adjusting sensitivity based on the average sensing value and average glucose level illustrated in FIG. 27.

FIG. 29 is a flowchart illustrating a first example of a method of calibrating sensitivity illustrated in FIG. 24 according to another embodiment.

FIG. 30 is a flowchart illustrating a second example of the method of calibrating sensitivity illustrated in FIG. 24 according to another embodiment.

FIG. 31 is a flowchart illustrating a third example of the method of calibrating sensitivity illustrated in FIG. 24 according to another embodiment.

FIG. 32 is a flowchart illustrating the method of calibrating sensitivity according to another embodiment.

## DETAILED DESCRIPTION OF THE INVENTION

The present invention may be variously modified and have several embodiments, and thus, specific embodiments will be illustrated in the drawings and be described. However, it is to be understood that the present invention is not limited to a specific embodiment, but includes all modifications, equivalents, and substitutions without departing from the scope and spirit of the present invention.

The terms including ordinal numbers such as 'second' and 'first' may be used to describe various components, but these components are not limited by these terms. The terms are used to distinguish one component from another component. For example, the second component may be named the first component and the first component may also be similarly named the second component, without departing from the scope of the present invention. A term 'and/or' includes a combination of multiple related described items or any one of the plurality of related described items.

It is to be understood that when one component is referred to as being "connected to" or "coupled to" another component, one component may be connected directly to or coupled directly to another component or be connected to or coupled to another component with the other component interposed therebetween. On the other hand, it is to be understood that when one component is referred to as being "connected directly to" or "coupled directly to" another component, it may be connected to or coupled to another component without the other component interposed therebetween.

The terms used herein are used only in order to describe specific embodiments rather than limiting the present invention. Singular forms include plural forms unless the context clearly indicates otherwise. It is to be understood that the term "include" or "have" used here specifies the presence of features, numbers, steps, operations, components, parts, or combinations thereof mentioned in the present specification, or combinations thereof, but does not preclude the presence or addition of one or more other features, numbers, steps, operations, components, parts, or combinations thereof.

Unless indicated otherwise, it is to be understood that all the terms used in the specification including technical and scientific terms have the same meaning as those that are generally understood by those who skilled in the art. Terms generally used and defined by a dictionary should be interpreted as having the same meanings as meanings within a context of the related art and should not be interpreted as having ideal or excessively formal meanings unless being clearly defined otherwise in the present specification.

Hereinafter, embodiments will be described in detail with reference to the accompanying drawings. The same reference numerals will be used to describe the same or similar components, independent of the reference numerals and an overlapped description of the same components will be omitted.

Hereinafter, a system for monitoring glucose will be described with reference to the drawings. The system for monitoring glucose described below may be a continuous glucose monitoring system that continuously or constantly measures and provides a glucose concentration of a subject. In the present invention, a continuous glucose measurement system that continuously measures biometric information indicating a glucose level through a body attachment unit attached to a user's body for a certain period and transmits the measured biometric information to a communication terminal will be described.

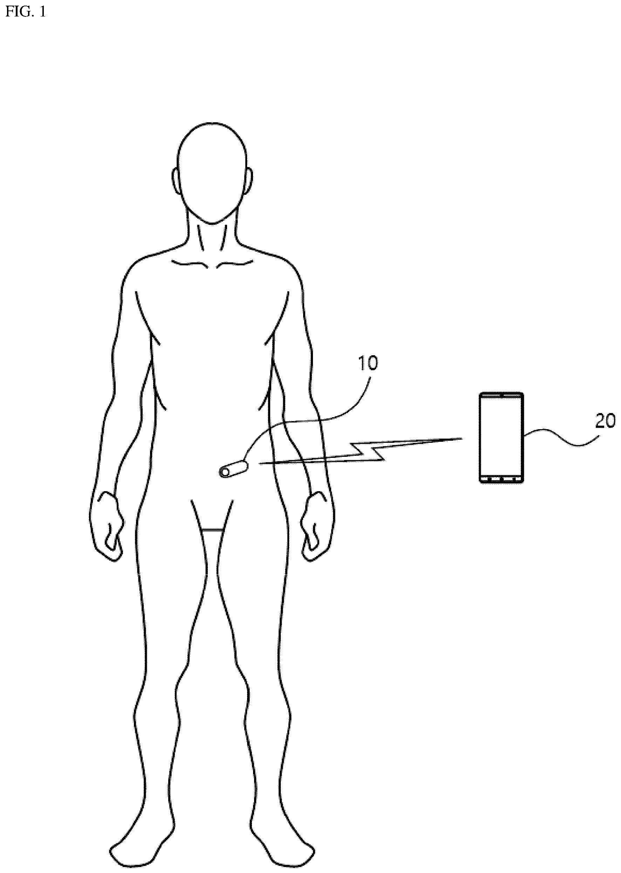

FIG. 1 is a diagram for describing a system for monitoring glucose according to an embodiment of the present invention.

Referring to FIG. 1, a system for monitoring glucose according to an embodiment of the present invention may include a sensor transmitter 10 and a receiver 20.

The sensor transmitter 10 may be attached to a human body B. When the sensor transmitter 10 is attached to the human body B, one end of the sensor transmitter 10 may be inserted into a skin to periodically measure a glucose concentration from a body fluid of the human body. In other words, the sensor may measure the glucose concentration in the body fluid through one end inserted into the skin. In this case, the body fluid of the human body may be interstitial fluid (ISF). The sensor transmitter 10 may convert the glucose concentration in the body fluid measured by the sensor into an electrical signal. The sensor transmitter 10 may convert the glucose concentration in the measured body fluid into biometric information and output the biometric information.

To perform this function, the sensor transmitter 10 may include a sensor module and a transmitter. According to an embodiment, the sensor transmitter 10 may implement the sensor module and the transmitter as an integrated structure. In another embodiment, the sensor transmitter 10 may implement the sensor module and the transmitter as a separate structure. In this case, the sensor module may be attached to the human body and then coupled to the transmitter.

First, the sensor module may be a device that is inserted into the human body and measures biometric information. The sensor module may be partially inserted into the skin and may measure the glucose concentration in the body fluid through the inserted portion.

Specifically, the sensor module may cause an electrochemical reaction with glucose contained in the interstitial fluid through a portion inserted into the skin. For example, the sensor module may include glucose oxidase, and contact the glucose contained in the interstitial fluid to allow the glucose oxidase to oxidize the glucose, thereby generating hydrogen peroxide ($H_2O_2$). The hydrogen peroxide may cause an oxidation-reduction reaction with an electrode included in the sensor module to generate current. The intensity of current generated in this process may correspond to the glucose concentration, so the sensor module may generate an electrical signal related to the glucose concentration in the interstitial fluid. The electrical signal may include a current value as an analog signal. In another embodiment, the electrical signal may include a voltage value as an analog signal.

Next, the transmitter may perform a function of processing the electrical signal generated by the sensor module. The transmitter may apply a set signal processing process to the electrical signal generated by the sensor module to generate the biometric information. The transmitter may transmit the generated biometric information to the receiver 20. To this end, the transmitter may include at least one of a control module, a communication module and a storage module.

The control module may control the overall configuration of the sensor transmitter 10 that includes the sensor module, the storage module, and the communication module.

According to an embodiment, the control module may process/manipulate an electrical signal. The control module may convert the electrical signal generated by the sensor module into the biometric information. The control module may convert the electrical signal, which is an analog signal, into the biometric information which is a digital signal. To this end, the control module may include an analog-to-digital converter (ADC). For example, the control module may amplify the electrical signal, and then convert the amplified electrical signal into the biometric information including a digital value through sampling, quantization, and coding steps. In addition, the control module may remove noise from the electrical signal before converting the electrical signal into the biometric information, if necessary. To this end, the control module may include a filter circuit, such as a low pass filter.

The communication module may transmit and receive various types of information to and from an external device.

According to an embodiment, the communication module may receive control information from the receiver 20. For example, the communication module may receive control information related to a transmission period of the biometric information from the receiver 20. The communication module may receive biometric information transmission request information from the receiver 20 or information on a transmission time (or time interval) of the biometric information.

According to an embodiment, the communication module may transmit the biometric information generated by the control module. The communication module may transmit the biometric information to the receiver 20. In addition, the communication module may transmit the biometric information to a server through a communication relay such as a router.

In addition to the embodiment, the communication module may transmit and receive various data related to the system for monitoring glucose to and from the external device.

According to an embodiment, the communication module may include a set communication interface to communicate with the external device. For example, the communication module may include a wired communication interface such as a USB communication module to perform wired communication with the receiver 20. Alternatively, the communication module may include a wireless communication interface, such as an infrared communication, NFC communication, Bluetooth, or WiFi communication module to perform wireless communication with the external device such as the receiver 20 or the server.

The storage module may store various types of information.

According to an embodiment, the storage module may store the biometric information generated by the control module. The storage module may store the biometric information or delete the stored biometric information under control of the control module.

According to an embodiment, the storage module may store control information. The storage module may store control information set at a factory. The storage module may store the control information received from the external device such as the receiver 20 or the server.

The receiver 20 may provide a user with various types of information based on the biometric information received from the sensor transmitter 10 and perform data processing accordingly.

According to an embodiment, the receiver 20 may acquire a glucose concentration contained in blood from the biometric information including the glucose information in the body fluid using a set algorithm. The set algorithm may be stored in the receiver 20. The receiver 20 may provide the acquired blood glucose concentration to the user through an output means such as a display.

According to an embodiment, the receiver 20 may generate various types of information based on the acquired glucose concentration in the blood. For example, the receiver 20 may generate trend information of the glucose concentration in the blood over a set time. Alternatively, the receiver 20 may generate warning information, such as low glucose warning or high glucose warning, based on the glucose concentration in the blood. The receiver 20 may provide the generated warning information to the user through the output means, such as the display or speaker.

In addition to the above embodiment, the receiver 20 may generate various types of information based on the biometric information and provide the generated information to a user.

In order to perform the above function, the receiver 20 may be implemented as various types of devices having a computational function, a storage function, and an output function. For example, the receiver 20 may include various devices, such as a smartphone, a mobile phone, a tablet PC, a desktop, and a laptop.

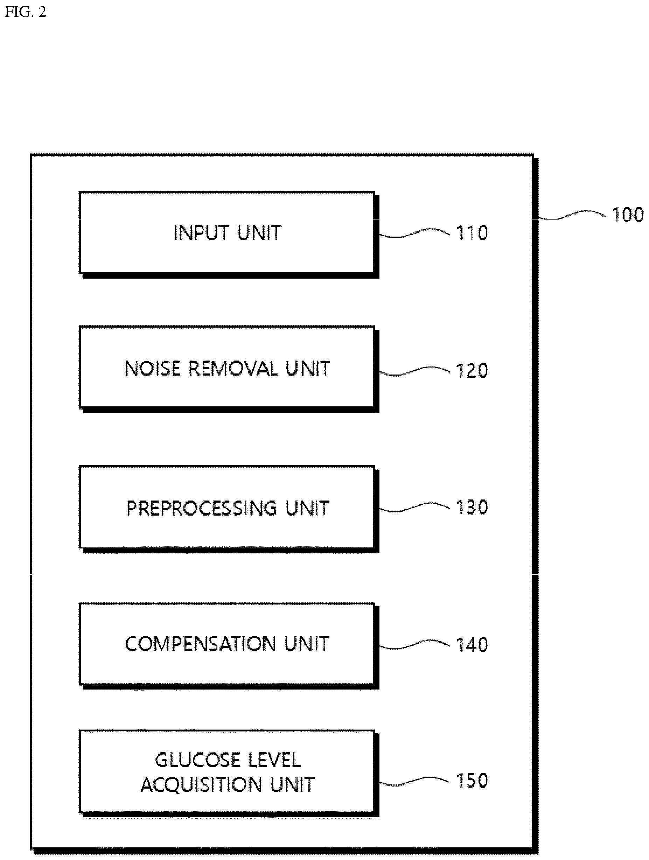

FIG. 2 is a configuration diagram of an apparatus for monitoring glucose according to an embodiment of the present invention.

An apparatus 100 for monitoring glucose described below refers to a device that acquires a glucose concentration, which is a glucose concentration in blood, from the biometric information including a glucose concentration in a body fluid. According to an embodiment, the apparatus 100 for monitoring glucose may be implemented through the receiver 20. According to another embodiment, the apparatus 100 for monitoring glucose may be implemented through the sensor transmitter 10. According to another embodiment, the apparatus 100 for monitoring glucose may be implemented through the sensor transmitter 10 and the receiver 20. That is, some functions of the apparatus 100 for monitoring glucose may be implemented through the sensor transmitter 10, and some functions may be implemented through the receiver 20.

Referring to FIG. 2, the apparatus 100 for monitoring glucose according to an embodiment of the present invention may include an input unit 110, a noise removal unit 120, a preprocessing unit 130, a compensation unit 140, and a glucose level acquisition unit 150.

First, the input unit 110 may obtain the biometric information measured by the sensor module. The input unit 110 may obtain the biometric information including a glucose concentration in a body fluid of a subject measured by the sensor module.

According to an embodiment, first biometric information including a plurality of data points measuring glucose-related information of a subject may be obtained.

Here, the glucose-related information of the subject may be glucose concentration information in the body fluid of the subject. For example, the glucose-related information of the subject may mean information including a current value related to a glucose concentration in a body fluid (such as interstitial fluid) of a person to be measured.

Here, the data point may mean an individual current value for the glucose concentration in the body fluid measured by the sensor module. For example, the sensor module may measure the glucose concentration in the body fluid at 10-second intervals. In this case, the data point may be generated at 10-second intervals. In this case, the plurality of data points may be a plurality of current values representing the glucose concentration in the plurality of body fluids measured at 10-second intervals.

Here, the first biometric information may mean the biometric information measured by the sensor module. Specifically, the first biometric information may be information that converts an analog signal including a current value measured by the sensor module into a digital signal through an analog-to-digital converter. In this case, the analog signal may not undergo separate signal processing, but is not limited thereto. The analog signal may undergo a set signal processing process, such as noise removal, and the first biometric information may be generated by converting the analog signal, which has undergone the set signal processing process, into the digital signal. That is, the first biometric information may be information representing the glucose concentration in the body fluid of the subject, and may mean information that does not undergo separate data processing in the apparatus 100 for monitoring glucose.

Next, the noise removal unit 120 may perform a function of removing noise from the biometric information.

According to an embodiment, the noise removal unit 120 may remove noise information included in the first biometric information. Here, the noise information may include information on various outliers included in the biometric information. For example, the noise information may include information caused by electrical interference occurring in the external device or inside the body. The noise information may include information caused by external environmental factors such as ambient temperature, humidity, and body temperature increase due to a user's exercise. The noise information may include information caused by user's physical movement. The noise information may include information caused by an unstable power status of the sensor transmitter 10. In addition, the noise information may include information caused by other factors, not the glucose concentration in the body fluid.

According to an embodiment, the noise removal unit 120 may perform a first noise removal process and a second noise removal process.

Regarding the first noise removal process, the noise removal unit 120 may perform the first noise removal process of removing a data point determined as noise information among first data points based on a plurality of data points included in a set time interval.

The noise removal unit 120 may perform the first noise removal process including the following steps. First, the noise removal unit 120 may determine whether any one of the data points is an outlier by using data points other than any one of the plurality of data points included in the set time interval. For example, the noise removal unit 120 may acquire a reference value including at least one of an average slope, a slope change value, an average value, and a standard deviation by using the data points other than any one of the plurality of data points. The noise removal unit 120 may compare the reference value with one data point to determine whether one data point is an outlier.

The noise removal unit 120 may remove any one of the data points when it is determined that any one of the data points is the outlier.

Regarding the second noise removal process, the noise removal unit 120 may perform the second noise removal process of adjusting a value of the first data point based on the plurality of data points included in the set time interval to remove set noise information.

The noise removal unit 120 may perform the second noise removal process including the following steps. First, the noise removal unit 120 may acquire a weight for any one of the data points by using the data points other than any one of the plurality of data points.

The noise removal unit 120 may apply the weight to any one of the data points to remove a noise component included in any one of the data points.

Next, the preprocessing unit 130 may perform a function of processing the biometric information from which noise has been removed.

According to an embodiment, the preprocessing unit 130 may preprocess the first biometric information from which the noise information has been removed to generate second biometric information having a lower sampling rate than the first biometric information.

According to an embodiment, the preprocessing unit 130 may perform a first preprocessing process of preprocessing the first biometric information from which the noise information has been removed and the first biometric information from which the noise information has been removed based on a first time interval. Describing the first preprocessing process in detail, first, the preprocessing unit 130 may divide the plurality of data points included in the first biometric information from which the noise information has been removed at the first time interval. The preprocessing unit 130 may remove the first data point related to a set upper value and a second data point related to a set lower value among the plurality of data points at each first time interval. The preprocessing unit 130 may acquire an average value of the plurality of data points from which the first data point and the second data point have been removed at each first time interval as first preprocessing data.

According to an embodiment, the preprocessing unit 130 may perform a second preprocessing process of preprocessing the first preprocessing data based on the first preprocessing data and a second time interval having a value greater than the first time interval. Describing the second preprocessing process in detail, first, the preprocessing unit 130 may divide a plurality of average values included in the first preprocessing data at the second time interval. The preprocessing unit 130 may remove a first average value related to a set upper value and a second average value related to a set lower value among the plurality of average values at each second time interval. The preprocessing unit 130 may acquire the average value of the plurality of average values from which the first average value and the second average value are removed as the second preprocessing data at each second time interval. The preprocessing unit 130 may determine the second preprocessing data as the second biometric information.

Next, the compensation unit 140 may perform a function of generating a calibration algorithm that may convert the biometric information into a glucose level based on the biometric information.

According to an embodiment, the compensation unit 140 may generate compensation data based on the second biometric information.

According to an embodiment, the compensation unit 140 may acquire an average value of data points included in the second biometric information for a set period. The compensation unit 140 may apply the average value to the second biometric information to generate ideal data having a set baseline. The compensation unit 140 may generate a first weight and a second weight. Here, the first weight and the second weight may have a negative correlation. The compensation unit 140 may add up a value obtained by applying the first weight to the second biometric information and a value obtained by applying the second weight to the ideal data to generate the compensation data.

According to an embodiment, the compensation unit 140 may determine any one of values of the pre-stored table data as an offset value based on the second biometric information. The compensation unit 140 may apply the offset value to the second biometric information to generate the compensation data.

According to an embodiment, the compensation unit 140 may generate a calibration algorithm based on the compensation data.

According to an embodiment, the compensation unit 140 may determine a first sensitivity based on the compensation data and a set reference glucose level. The compensation unit 140 may adjust the first sensitivity to determine a final sensitivity.

According to an embodiment, the compensation unit 140 may perform at least one sensor sensitivity determination process and determine one of a second sensitivity, a third sensitivity, and a fourth sensitivity as the final sensitivity.

The compensation unit 140 may perform the first sensor sensitivity determination process of adjusting the first sensitivity based on the sensor sensitivity set in response to the glucose measurement sensor and determining the adjusted first sensitivity as the second sensitivity. Describing the first sensor sensitivity determination process in detail, first, the compensation unit 140 may determine the sensor sensitivity as the second sensitivity when the first sensitivity is higher than the sensor sensitivity. The compensation unit 140 may determine the sensor sensitivity, which is processed through a set algorithm, as the second sensitivity when the first sensitivity is lower than the sensor sensitivity. On the other hand, the compensation unit 140 may determine the first sensitivity as the second sensitivity when the first sensitivity is equal to the sensor sensitivity.

The compensation unit 140 may perform a second sensor sensitivity determination process of adjusting the second sensitivity based on a first previous time point sensitivity and a first previous time point final sensitivity that are determined at a previous time point and determining the adjusted second sensitivity as the third sensitivity. Describing the second sensor sensitivity determination process in detail, the compensation unit 140 may determine an average value of the first sensitivity and the second sensitivity as the third sensitivity when the state in which the first sensitivity determined at the previous time point is higher or lower than the final sensitivity at corresponding previous time point continuously occurs.

The compensation unit 140 may adjust the third sensitivity according to whether the glucose level acquired based on the third sensitivity falls within a set range, and determine the adjusted third sensitivity as the fourth sensitivity. Describing the third sensor sensitivity determination process in detail, first, the compensation unit 140 may acquire the glucose level related to the compensation data using the third sensitivity. The compensation unit 140 may determine the third sensitivity as the fourth sensitivity when the glucose level falls within a preset range. On the other hand, the compensation unit 140 may determine the fourth sensitivity based on the glucose level at the previous time point, the reference glucose level, and the compensation data when the glucose level does not fall within the preset range.

Next, the glucose level acquisition unit 150 may perform a function of acquiring the glucose level based on the generated calibration algorithm and the biometric information.

According to an embodiment, the glucose level acquisition unit 150 may acquire the glucose level related to the second biometric information by reflecting the calibration algorithm and a set time delay.

According to an embodiment, the glucose level acquisition unit 150 may compensate for the time delay of the second biometric information to generate time-compensated biometric information.

Here, the time delay may mean a physiological time delay. The glucose concentration measured from a body fluid such as interstitial fluid (ISF) of a human body has a time delay compared to the glucose concentration measured from blood of a human body. The reason why the time delay occurs in the glucose concentration between the ISF and the blood is because a physical and physiological delay occurs in the process in which the glucose in the blood diffuses into the ISF. The causes of physical and physiological delays include various influences such as differences in a diffusion speed of glucose in cells and capillaries, permeability of cell membranes, and speed of blood circulation. In addition, the time delay may mean various time delays in addition to the physiological time delay. The time delay may include a time delay due to the physical structure of the system for monitoring glucose itself, such as the sensor transmitter and the receiver, a time delay due to a computational time required to acquire the biometric information from the sensor signal, a time delay that occurs between the time when the sensor transmitter actually measures the user's biometric information and the time when the communication terminal obtains the user's biometric information, etc. The time delay compensated by the glucose level acquisition unit 150 may be the time delay due to any one of the causes, but is not limited thereto. The time delay reflected by the glucose level acquisition unit 150 may be the time delay that is a combination of various causes described above.

Specifically, the glucose level acquisition unit 150 may compensate for the time delay in the second biometric information based on the prediction algorithm to generate the time-compensated biometric information. Therefore, the glucose level acquisition unit 150 may generate the time-compensated biometric information that may represent accurate glucose information at each time point by reflecting the time delay to the second biometric information. Here, the prediction algorithm may include a Kalman filter, a linear adaptive model, an autoregressive integrated moving average model (ARIMA model), a linear regression algorithm, a moving average and weighted moving average, Bayesian filtering, a machine learning model, etc.

The glucose level acquisition unit 150 may apply the time-compensated biometric information to the calibration algorithm to acquire the glucose level.

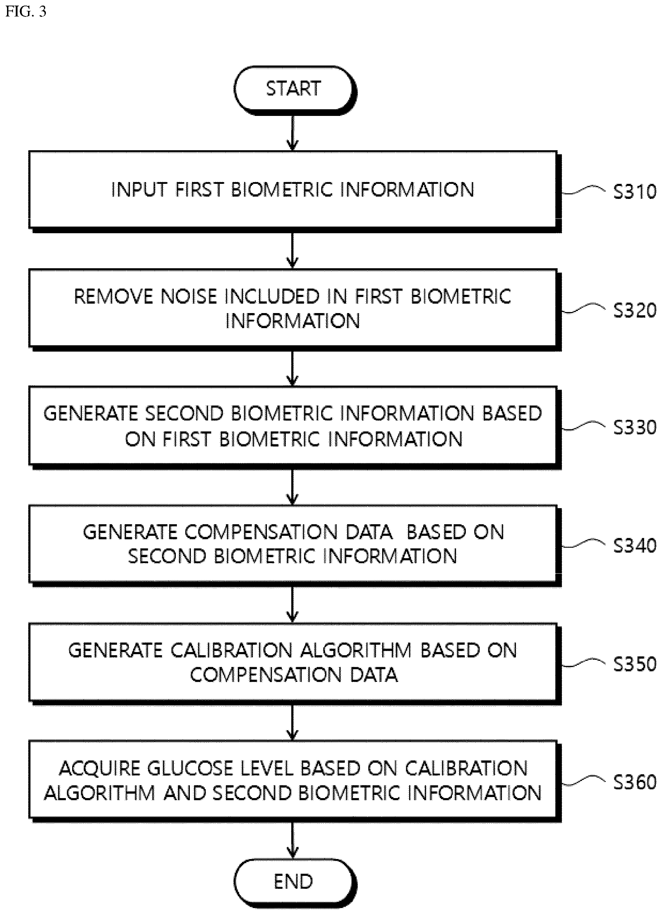

FIG. 3 is a flowchart of a method of monitoring glucose according to an embodiment of the present invention.

Referring to FIG. 3, a method of monitoring glucose using an apparatus 100 for monitoring glucose according to an embodiment of the present invention may include steps S310 to S360.

First, the input unit 110 may obtain the first biometric information including a plurality of data points measuring glucose-related information of a subject (S310).

Next, the noise removal unit 120 may remove the noise information included in the first biometric information (S320).

Next, the preprocessing unit 130 may preprocess the first biometric information from which the noise information has been removed to generate the second biometric information having a lower sampling rate than the first biometric information (S330).

Next, the compensation unit 140 may generate the compensation data based on the second biometric information (S340).

Next, the compensation unit 140 may generate the calibration algorithm based on the compensation data (S350).

Next, the glucose level acquisition unit 150 may acquire a glucose level related to the second biometric information by reflecting the calibration algorithm and a set time delay (S360).

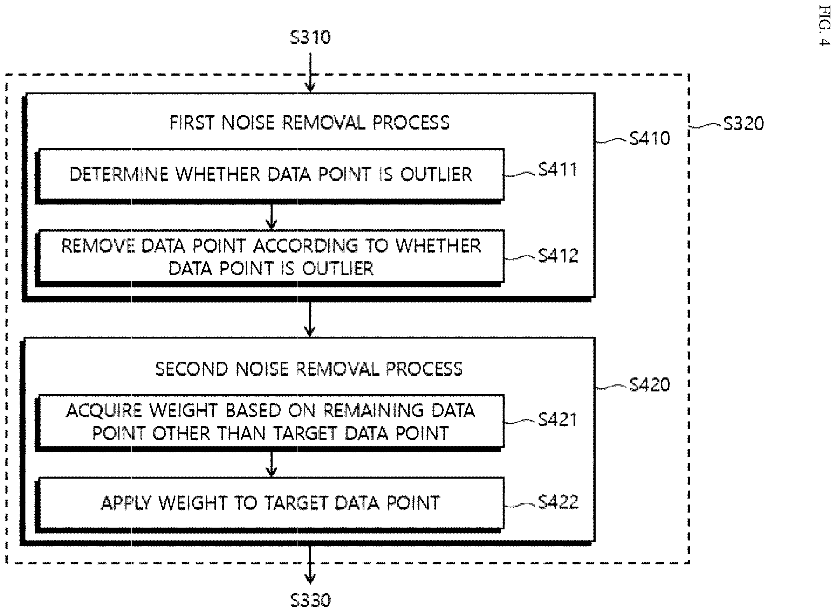

Hereinafter, step S320 performed by the noise removal unit 120 of the present invention will be described in detail with reference to FIGS. 4 to 6.

FIG. 4 is a flowchart illustrating in detail step S320 of FIG. 3.

Referring to FIGS. 3 and 4, the apparatus 100 for monitoring glucose according to an embodiment of the present invention may remove the noise information included in the first biometric information.

The signal measured by the sensor may include various types of noise information such as random noise, environmental noise, system noise, interference noise, stationary noise, spy noise, and Gaussian noise. Therefore, it is necessary to appropriately separate and remove such noise. For example, the spy noise may be considered as an outlier, and a noise filter suitable for the spy noise should be used to efficiently and accurately remove noise information from the signal. As another example, the random noise, the stationary noise, and the Gaussian noise generated due to irregular fluctuations in the external environment or system should be removed efficiently and accurately from a signal by using a noise filter that uses noise filtering based on statistical characteristics. According to an embodiment, the apparatus 100 for monitoring glucose may perform at least one of outlier processing filtering, low pass filtering, preprocessing, and linear regression filtering on the first biometric information to process noise.

Referring back to FIG. 4, the noise removal unit 120 may perform the first noise removal process of removing the data points determined as the noise information among the first data points based on the plurality of data points included in the set time interval (S410). For example, the first noise removal process may include an outlier processing filtering process, which may be a process of removing data points that deviate from a set condition.

Specifically, the first noise removal process may include steps S411 and S412.

First, the noise removal unit 120 may determine whether any one of the data points is an outlier by using data points other than any one of the plurality of data points included in the set time interval (S411). According to an embodiment, the noise removal unit 120 may acquire a reference value including at least one of an average slope, a slope change value, an average value, and a standard deviation by using the data points other than any one of the plurality of data points. The noise removal unit 120 may compare the reference value with one data point to determine whether one data point is an outlier.

The noise removal unit 120 may remove any one of the data points when any one of the data points is determined as the outlier (S412).

Next, the noise removal unit 120 may adjust the value of the first data point based on the plurality of data points included in the set time interval to perform the second noise removal process of removing the set noise information. The second noise removal process may be low pass filtering. The low pass filtering process may be a process of removing noise components by removing components related to a high pass and leaving only data related to a low pass. Specifically, the noise removal unit 120 may transform the value of the data using the plurality of data based on any one of the plurality of data points. Therefore, when the glucose information data is transformed through low pass filtering in this way, the transformed glucose information data may be recognized as reliable data.

First, the noise removal unit 120 may acquire a weight for any one of the data points by using the data points other than any one of the plurality of data points (S421).

The noise removal unit 120 may apply the weight to one of the data points to remove a noise component included in any one of the data points (S422).

In FIG. 4, for the convenience of description, the second noise removal process is performed after the first noise removal process, but the present invention is not limited thereto.

According to an embodiment, the second noise removal process may be performed prior to the first noise removal process. According to an embodiment, the first noise removal process and the second noise removal process may be performed substantially simultaneously. According to an embodiment, any one of the first noise removal process or the second noise removal process may be selectively performed.

According to an embodiment, the second noise removal process may be performed under the set condition. For example, the noise removal unit 120 may determine whether the plurality of data points on which the first noise removal process has been performed are reliable. The noise removal unit 120 may generate verification data using the glucose information data that has undergone the first noise removal process, and when the generated verification data falls within the set range, may determine the data point that has undergone the first noise removal process to be reliable. In this case, the data processing process such as a truncated average value may be performed on the plurality of data points that have undergone the first noise removal process, and the noise removal unit 120 may determine whether the plurality of data points on which such data processing process has been performed are reliable. When the data point is not recognized as a reliable data point, the noise removal unit 120 may perform the second noise removal process on the data point that has undergone the first noise removal process.

Although not illustrated in the drawing, the noise removal unit 120 may also perform linear regression filtering on the plurality of data points. The linear regression filtering may be performed using the low-pass filtered data points. The linear regression filtering may perform the filtering on the corresponding data by using a plurality of previous data based on one data. For example, when it is assumed that the filtering is performed on B7 among data of B1 to B7, the filtering on B7 may be performed using data of B1 to B6.

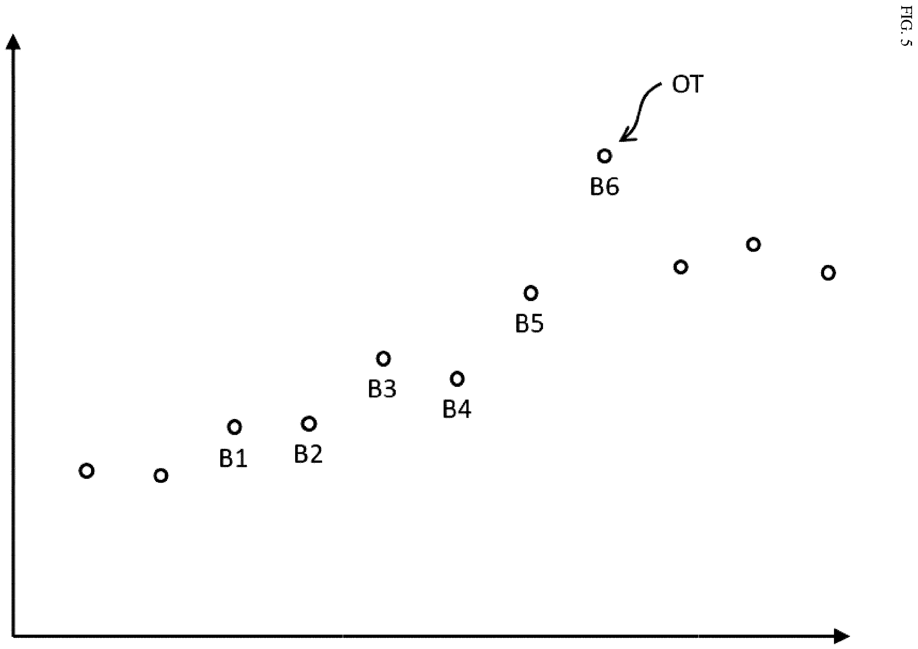

FIG. 5 is a diagram for describing an outlier filtering process according to an embodiment of the present invention.

A process of processing an outlier for noise processing on glucose information measurement data, i.e., data points, obtained from the sensor transmitter 10 will be described with reference to FIG. 5. The noise removal unit 120 determines an outlier included in a data point and processes the determined outlier. In this embodiment, the determined outlier is removed and processed.

As illustrated, to determine whether data has an outlier OT, the noise removal unit 120 determines whether the data point has the outlier by using the plurality of previous data points based on one data point. That is, data points of B1 to B5 are used to determine whether B6 among data points of B1 to B6 is an outlier.

In this case, an average slope of B1 to B5 may be used to determine whether B6 is an outlier. When a value of B6 does not fall within the set range of an average slope of B1 to B5, the value may be determined as the outlier.

Alternatively, the slope change value of B1 to B5 may be used to determine whether B6 is an outlier. When a value of B6 does not fall within the set range of the slope change value of B1 to B5, the value may be determined as an outlier.

Alternatively, the average and standard deviation of B1 to B5 may be used to determine whether B6 is an outlier. Therefore, when the value of B6 does not fall within the standard deviation of B1 to B5, the value may be determined as an outlier.

When it is determined that B6 is an outlier, the corresponding data point may be removed and processed. However, B6 is not limited thereto, and when necessary, B6 having an outlier may be corrected and used to be corrected so that B6 falls within the range of values of B1 to B5.

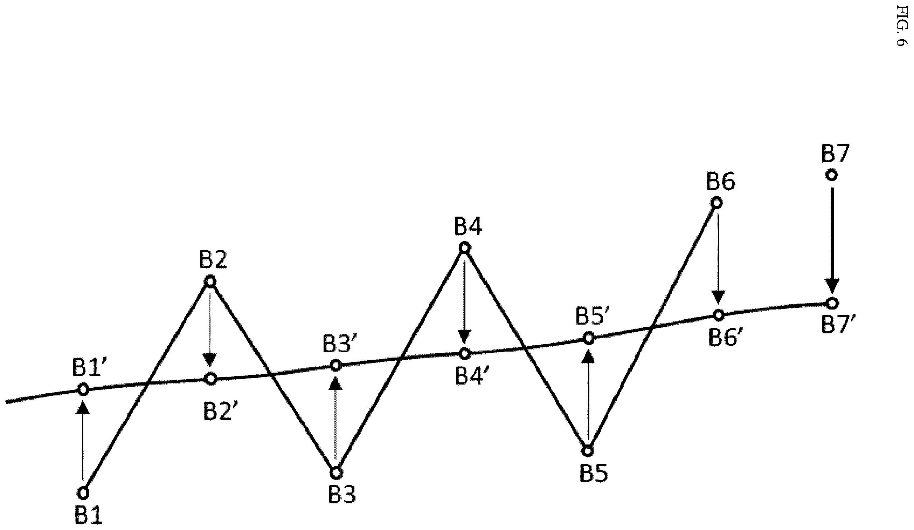

FIG. 6 is a diagram for describing a low pass filtering process according to an embodiment of the present invention.

Referring to FIG. 6, the low pass filtering process is performed by the noise removal unit 120 for noise processing on the data points for which the outliers are processed. The noise removal unit 120 removes components related to a high pass from data points and filters data points so that only data points related to a low pass remain.

To this end, the noise removal unit 120 may transform the values of each data by setting weights for each data for which outliers are processed. In order to set weights for each data point, the noise removal unit 120 sets the weight for the corresponding data using the plurality of previous data based on one data.

That is, as illustrated, a weight set for B7 among data of B1 to B7 may be determined by weights set for B1 to B6. As illustrated, the values of B1 to B6 are transformed into B1' to B6' by the weights set for each value of B1 to B6. Accordingly, the weight for B7 may be set using the values transformed from B 1' to B6'.

Therefore, when the value of B7 is greater than the values transformed into B1' to B6', the weight may be set to decrease the value, and when the value of B7 is smaller than the values transformed into B1' to B6', the weight may be set to increase the value. Therefore, the value of B7 may be transformed into B7'.

As described above, as the weights are set for each data point and transformed, the components related to the high pass may be removed.

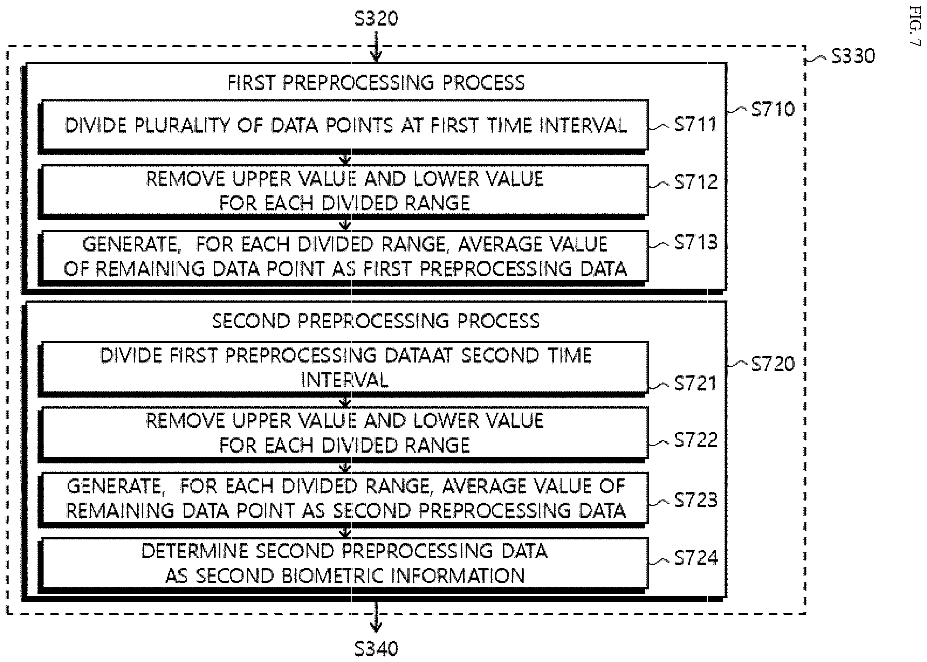

Hereinafter, step S330 performed by the preprocessing unit 130 of the present invention will be described in detail with reference to FIGS. 7 to 9.

FIG. 7 is a flowchart illustrating in detail step S330 of FIG. 3.

Referring to FIGS. 3 and 7, the apparatus 100 for monitoring glucose according to the embodiment of the present invention may perform the first preprocessing process and the second preprocessing process.

The preprocessing unit 130 may perform a first preprocessing process of preprocessing the first biometric information from which the noise information has been removed and the first biometric information from which the noise information has been removed based on the first time interval (S710). Here, the first preprocessing process may include steps S711 to S713.

Specifically, the preprocessing unit 130 may divide the plurality of data points included in the first biometric information from which the noise information has been removed at the first time interval (S711).

The preprocessing unit 130 may remove the first data point related to a set upper value and a second data point related to a set lower value among the plurality of data points at each first time interval (S712).

The first preprocessing unit 130 may acquire an average value of the plurality of data points from which the first data point and the second data point have been removed at each first time interval as first preprocessing data (S713).

The preprocessing unit 130 may perform a second preprocessing process of preprocessing the first preprocessing data based on the first preprocessing data and the second time interval having a value greater than the first time interval (S720). Here, the second preprocessing process may include steps S721 to S724.

Specifically, the preprocessing unit 130 may divide the plurality of average values included in the first preprocessing data at the second time interval (S721).

The preprocessing unit 130 may remove the first average value related to the set upper value and the second average value related to the set lower value among the plurality of average values at each second time interval (S722).

The preprocessing unit 130 may acquire the average value of the plurality of average values from which the first average value and the second average value are removed as the second preprocessing data at each second time interval (S723).

The preprocessing unit 130 may determine the second preprocessing data as the second biometric information (S724).

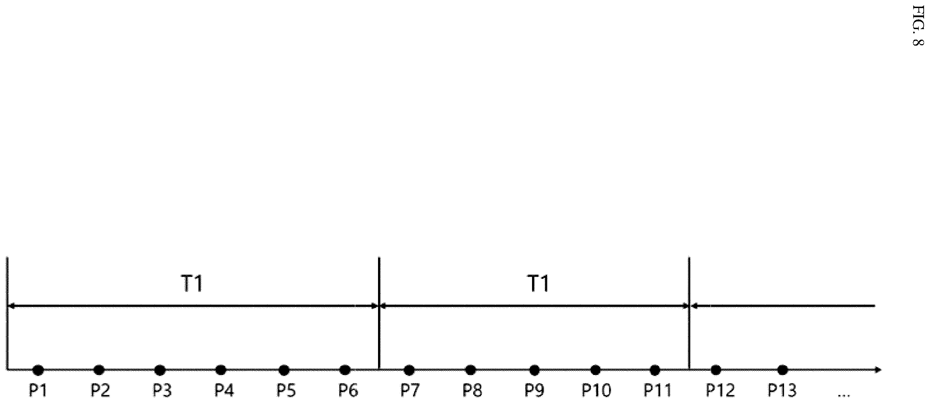

FIG. 8 is a diagram for describing a process of generating a first preprocessing process according to an embodiment of the present invention.

FIG. 8 illustrates the first biometric information from which the noise has been removed. Each point illustrated in the diagram represents the data point included in the first biometric information from which the noise has been removed.

According to an embodiment of the present invention, the preprocessing unit 130 may set a first time interval T1 to 1 minute to perform the first preprocessing process. For example, when it is assumed that the sensor measures data every 10 seconds, the first biometric information may include up to 6 data points every 1 minute. The first biometric information from which the noise information has been removed may include 6 or fewer data points every 1 minute. Accordingly, when the first biometric information is divided according to the first time interval T1, a first section may include 6 data points D1 to D6, and a second section may include 5 data points D7 to D11. The preprocessing unit 130 may search for an upper value and a lower value for the first section. When D1 to D6 included in the first section are searched as the upper value having the largest value and D6 is searched as the lower value having the smallest value, the preprocessing unit 130 may remove D1 and D6 from the first section.

That is, the first section includes four data points of D2 to D5. The preprocessing unit 130 acquires an average value of D2 to D5. In this case, the acquired average value becomes the first preprocessing data for the first section.

In addition, the first preprocessing data is also generated for the second section including five data points of D7 to D11. First, the preprocessing unit 130 may search for the upper value and the lower value for the second section. When D11 of D7 to D11 included in the second section are searched as the upper value having the largest value and D7 is searched as the lower value having the smallest value, the preprocessing unit 130 may remove D7 and D11 from the second section. That is, the first section includes three data points of D8 to D10. The preprocessing unit 130 acquires an average value of D8 to D10. In this case, the acquired average value becomes the first preprocessing data for the second section.

Even after the third section, the first preprocessing data is generated according to the steps described above.

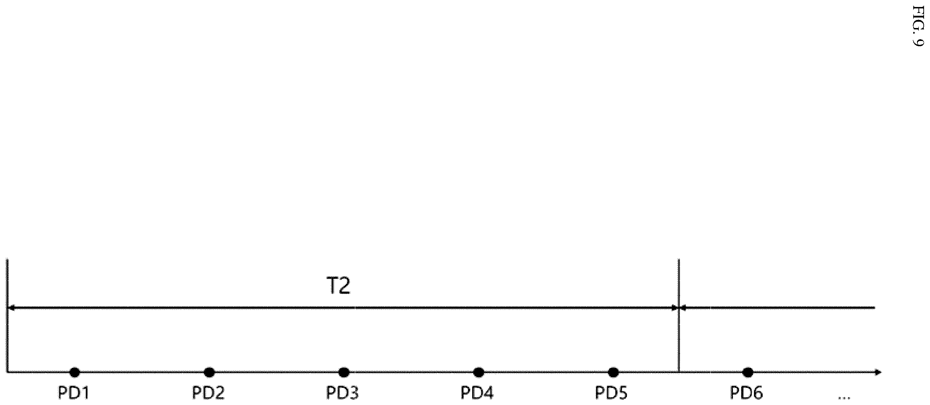

FIG. 9 is a diagram for describing a process of generating a second preprocessing process according to an embodiment of the present invention.

FIG. 9 illustrates the first biometric information, i.e., the first preprocessing data, on which the first preprocessing process is performed. Each point illustrated in the drawing represents the data point included in the first preprocessing data.

According to an embodiment of the present invention, the preprocessing unit 130 may set a second time interval T2 to 5 minutes to perform the second preprocessing process. For example, when the sensor measures data every 10 seconds and the first time interval is assumed to be 1 minute, the first preprocessing data may include up to 5 data points every 5 minutes. Accordingly, when the first preprocessing data is divided according to the first time interval T1, the first section may include 5 data points PD1 to PD5. The preprocessing unit 130 may search for the upper value and the lower value for the first section. When the PD1 is searched as the upper value having the largest value among PD1 to PD5 included in the first section, and the PD5 is searched as the lower value having the smallest value, the preprocessing unit 130 may remove the PD1 and PD5 from the first section. That is, the first section includes three data points of PD2 to PD3. The preprocessing unit 130 acquires an average value of PD2 to PD3. In this case, the acquired average value becomes the second preprocessing data for the first section. This process may be repeated after the second section including PD6. Thereafter, the preprocessing unit 130 may determine the second preprocessing data as the second biometric information.

When the data point interval of the first biometric information and the data point interval of the second biometric information are compared, it may be seen that the sampling rates are different. According to the above example, in the case of the first biometric information, the data points are generated every 10 seconds, whereas in the case of the second biometric information, the data points are generated every 5 minutes. In this way, according to the present invention, by generating the second biometric information having a lower sampling rate than the first biometric information through the set preprocessing process, it is possible to provide the sensing information that is less sensitive to extreme values, more stable, more reliable, and more robust.

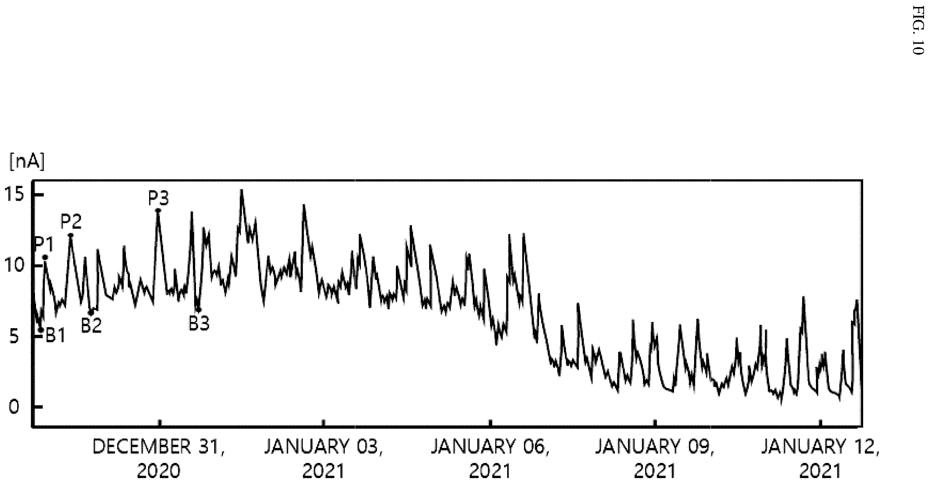

Hereinafter, the process of generating the compensation data by compensation unit 140 will be described in detail with reference to FIGS. 10 to 12.

First, a syllable fragmentation phenomenon of the sensor signal will be described with reference to FIGS. 10 to 12.

FIG. 10 is an exemplary diagram of an electrical signal measured by a sensor according to an embodiment.

The electrical signal described in FIG. 10 means a signal representing a glucose concentration in a body fluid measured by the sensor. The electrical signal may represent glucose information for several days (for example, for about 15 days) as a current value. The higher the glucose level of the subject, the higher the current value appears to form a peak, and the lower the glucose level, the lower the current value appears to form a bottom. The electrical signal may show the peak or bottom at a specific time point according to the glucose level for several days. In FIG. 9, P1, P2, and P3 may represent peak values, and B1, B2, and B3 may represent bottom values, respectively, and more peaks and bottom values may appear over time.

In FIG. 9, the electrical signal has a waveform with a current value starting from about 5 nA and up to 15 nA, and then has a waveform with a bottom value below 5 nA around January 6, 2021. Then, around January 9, 2021, the bottom value drops to around 0 nA, and then the lowered bottom value is maintained. In this way, the electrical signal may react according to the glucose level and show the peak and bottom at a specific point time.

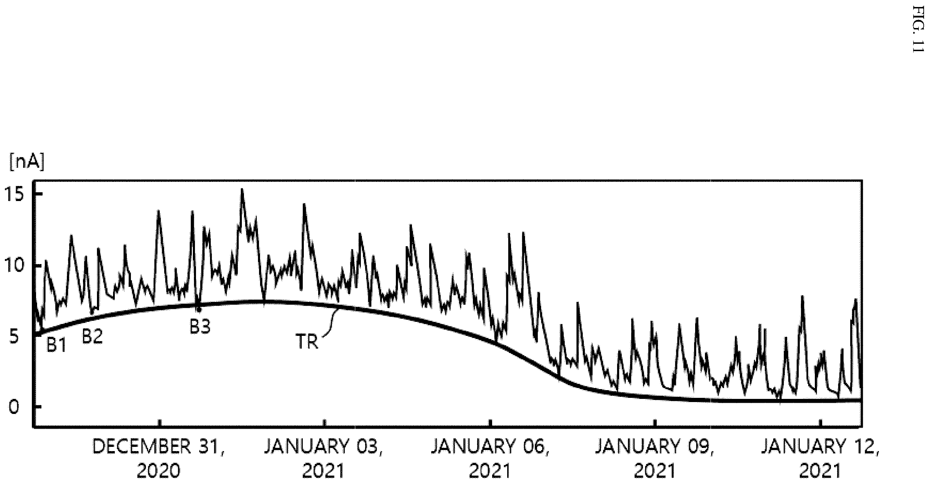

FIG. 11 is an exemplary diagram of an electrical signal showing a trend according to an embodiment.

Referring to FIG. 11, an example of an electrical signal representing a trend according to an embodiment may be illustrated. As described above, the electrical signal may have a curve until January 6, 2021, but have a somewhat constant bottom value. However, from January 6, 2021, the bottom value shows a decreasing trend, and from around January 9, 2021, the decreasing bottom value may be maintained at a constant level. In this way, when the bottom value changes due to the increase or decrease in the electrical signal, the trend may be formed. In FIG. 10, the trend may be represented as a line connecting the bottom values in the waveform of the electrical signal, and may be defined as TR. Since the current value of the electrical signal usually does not fall below the trend even when the glucose level decreases, a kind of baseline for the current value may be formed centered on the trend.

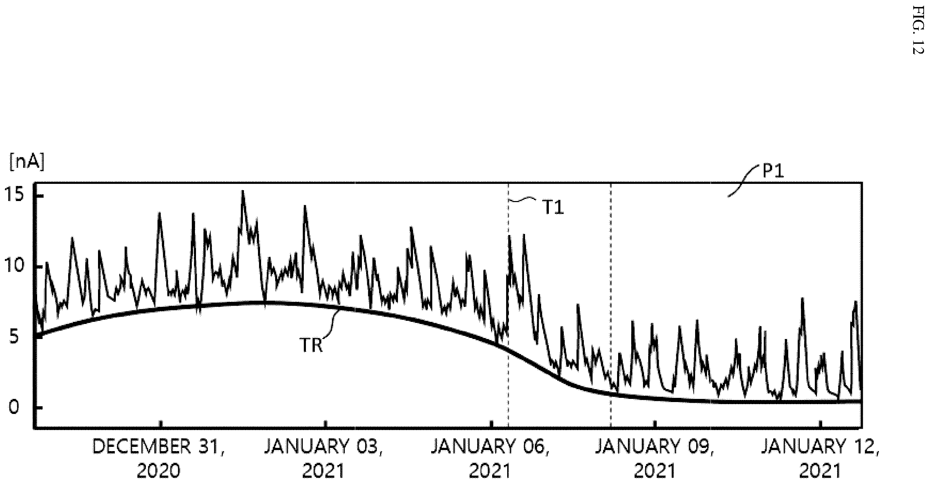

FIG. 12 is an exemplary diagram of a biosignal showing a syllable fragmentation phenomenon according to an embodiment.

Referring to FIG. 12, the syllable fragmentation phenomenon that appears in the electrical signal according to an embodiment may be illustrated. The syllable fragmentation phenomenon may be understood as the baseline decreases in conjunction with the lowering of the bottom value in the biosignal according to the glucose level. The bottom value that changes according to the glucose level generates the trend of the electrical signal, and the trajectory of the trend may gradually go down. Then, the baseline also gradually goes down, and may be maintained in the lowered state as it is. The phenomenon that the baseline decreases is called syllable fragmentation stabilization, and the section may be called a syllable fragmentation stabilization section.

For example, the electrical signal may begin to show the decreasing baseline -syllable fragmentation phenomenon- from time T1 around January 6, 2021. Then, the decreasing baseline may be maintained as it is from January 9, 2021 - the syllable fragmentation may be stabilized. The electrical signal may show the syllable fragmentation stabilization section during the section P1.

In this way, when the syllable fragmentation occurs and thus the syllable fragmentation is stabilized, a terminal for continuous glucose may output an inaccurate glucose level. This is because the current value decreases as the syllable fragmentation occurs, and thus, the low glucose may not be expressed properly. Of course, the terminal may show good sensitivity by showing the peak and bottom. However, the low glucose may be acquired as a lower glucose level and output to a user, or the terminal may acquire data based on the inaccurate current value to cause errors.

Accordingly, the present invention may use a fixed offset value or applying a time-varying weight to the electrical signal and the ideal signal to generate the compensated electrical signal, thereby solving the above problems.

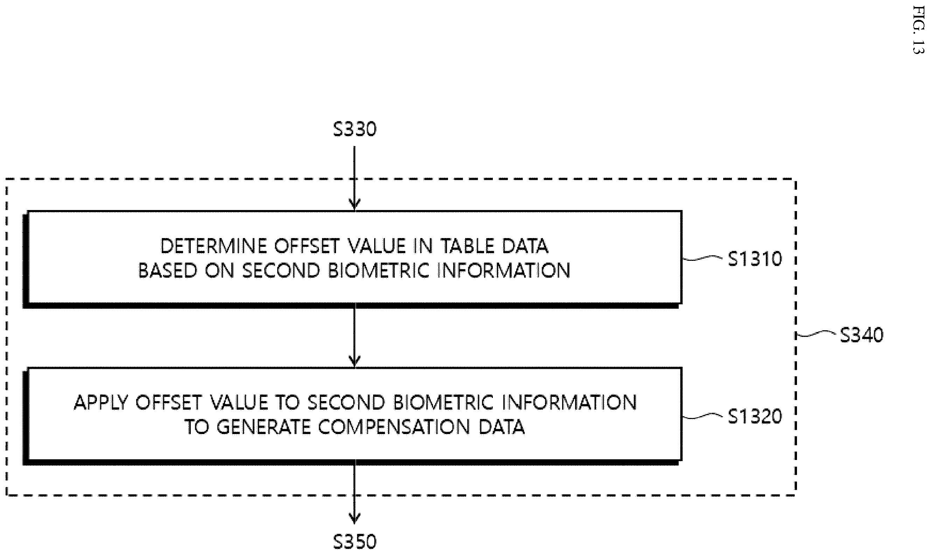

FIG. 13 is a flowchart of an embodiment illustrating in detail step S340 of FIG. 3.

Referring to FIGS. 3 and 13, the apparatus 100 for monitoring glucose according to an embodiment of the present invention may generate the compensation data based on the second biometric information.

Specifically, the compensation unit 140 may determine any one of the values of the pre-stored table data as an offset value based on the second biometric information (S1310). For example, the pre-stored table may be values set at a factory during the manufacturing of the sensor. As another example, the pre-stored table may be a value accumulated and stored while the user is using the sensor. In addition, the table data may be acquired and stored in various ways.

The compensation unit 140 may apply the offset value to the second biometric information to generate the compensation data (S1320).

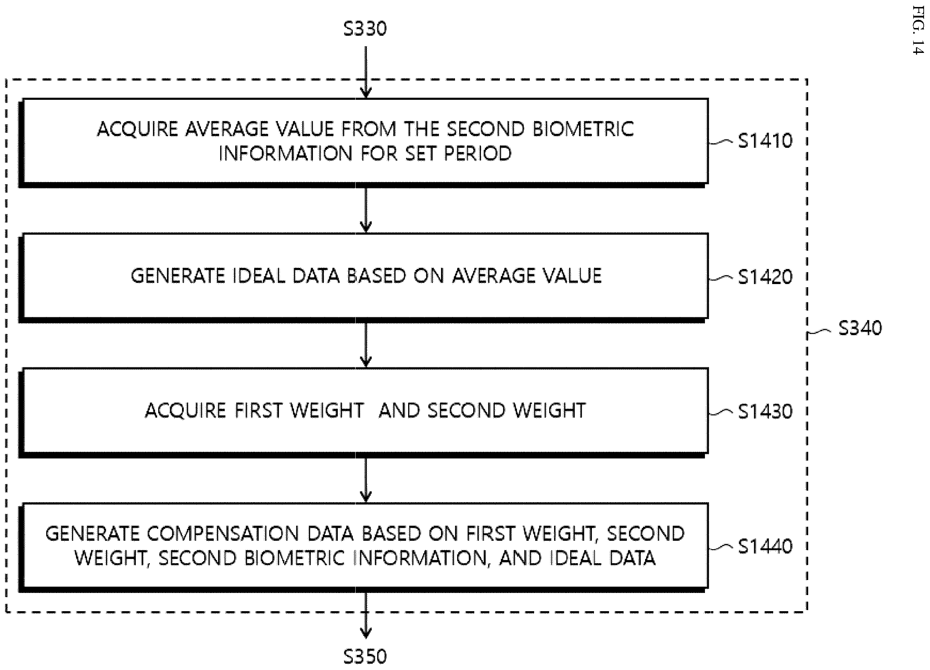

FIG. 14 is a flowchart of an embodiment illustrating in detail step S340 of FIG. 3.

Referring to FIG. 3 and FIG. 14, the apparatus 100 for monitoring glucose according to an embodiment of the present invention may generate the compensation data based on the second biometric information.

Specifically, the compensation unit 140 may acquire an average value of data points included in the second biometric information for a set period (S1410).

The compensation unit 140 may apply the average value to the second biometric information to generate ideal data having a set baseline (S1420). Specifically, the compensation unit 140 may remove the trend from the second biometric information to generate the ideal signal, that is, the ideal data, and apply the average value to the second biometric information from which the trend has been removed to generate the ideal signal. In this case, the compensation unit 140 may remove the trend of the second biometric information using a set filter. For example, the compensation unit 140 may include at least one of a low pass filter (LPF), a high pass filter (HPF), a band pass filter (BPF), and a band rejection filter (BRF) according to frequency characteristics. Alternatively, the compensation unit 140 may include a finite impulse response (FIR) filter or an infinite impulse response (IIR) filter as a digital filter. In this case, the compensation unit 140 may apply the second biometric information converted into a digital form to the digital filter.

The compensation unit 140 may generate the first weight and the second weight (S1430). Here, the first weight and the second weight may have a negative correlation.

The compensation unit 140 may add up a value obtained by applying the first weight to the second biometric information and a value obtained by applying the second weight to the ideal data to generate the compensation data (S1440). Specifically, the compensation unit 140 may generate a weight that vary over time and apply the weight to the second biometric information and the ideal signal, respectively. The compensation unit 140 may generate the compensated second biometric information as a result of the weight application. When the syllable fragmentation phenomenon is detected, the compensation unit 140 may apply the first weight and the second weight having the negative correlation to the second biometric information and the ideal signal, and add up the results to generate the compensation data which is the compensated second biometric information. When an intercept goes down to a negative value over time, the compensation unit 140 may also change the first weight and the second weight over time. Thereafter, the glucose level may be acquired from the compensated second biometric information. Therefore, as the syllable fragmentation phenomenon is removed, the terminal may output a more accurate glucose level to the user.

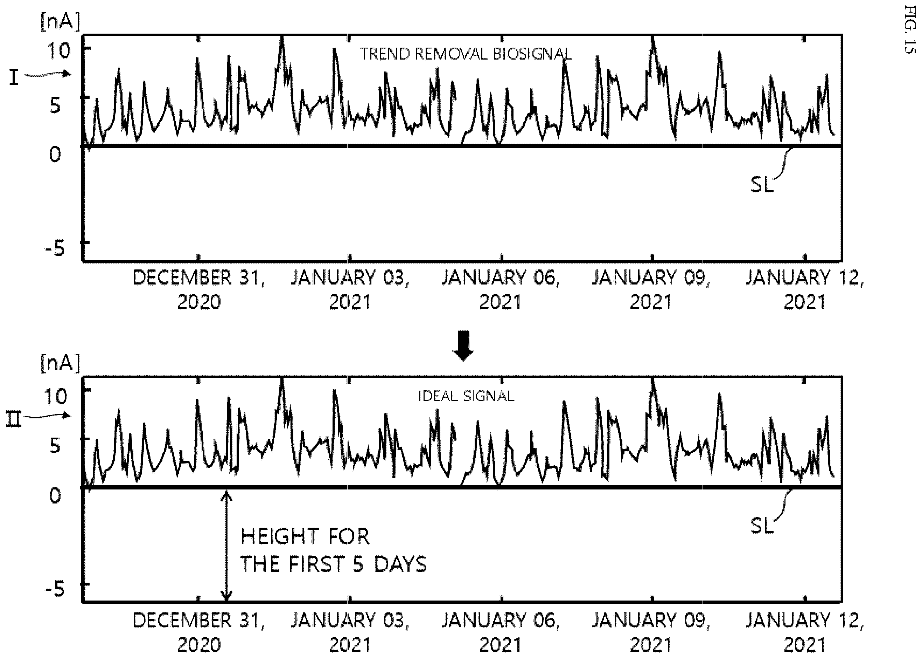

FIG. 15 is an exemplary diagram of an ideal signal from which the trend is removed and to which an average value is applied according to an embodiment of the present invention.

Referring to FIG. 15, the ideal data and its formation process may be illustrated. The apparatus 100 for monitoring glucose may remove the trend from the second biometric information and generate the second biometric information with a waveform I from which the trend has been removed. The second biometric information from which the trend has been removed may have generally similar bottom values. These bottom values may similarly converge to one line. Hereinafter, the line is defined as a baseline and may be illustrated as SL in FIG. 15. The baseline SL may have one value (e.g., a current value). Since the trend has been removed from the second biometric information, the reference value may be first formed as 0 (zero).

Next, the apparatus 100 for monitoring glucose may apply the average value to the second biometric information from which the trend has been removed to finally generate the ideal data. When the average value is applied to the second biometric information, the baseline SL may increase. In other words, the average value may increase a reference value. The reference value may increase from 0(zero) to 5 nA. The apparatus 100 for monitoring glucose may adjust the second biometric information from which the trend has been removed so that the ideal data (or its waveform) reaches a height of the waveform of the second biometric information formed for the first 5 days. Then, the apparatus 100 for monitoring glucose may finally generate an ideal data having a waveform (II). Here, the average value may be a result of sampling and averaging the current value of the second biometric information for a specific section before the syllable fragmentation occurs. For example, the apparatus 100 for monitoring glucose may sample the current values of the second biometric information for 5 days from December 29, 2020 to January 3, 2021 and apply the average value to the second biometric information from which the trend has been removed.

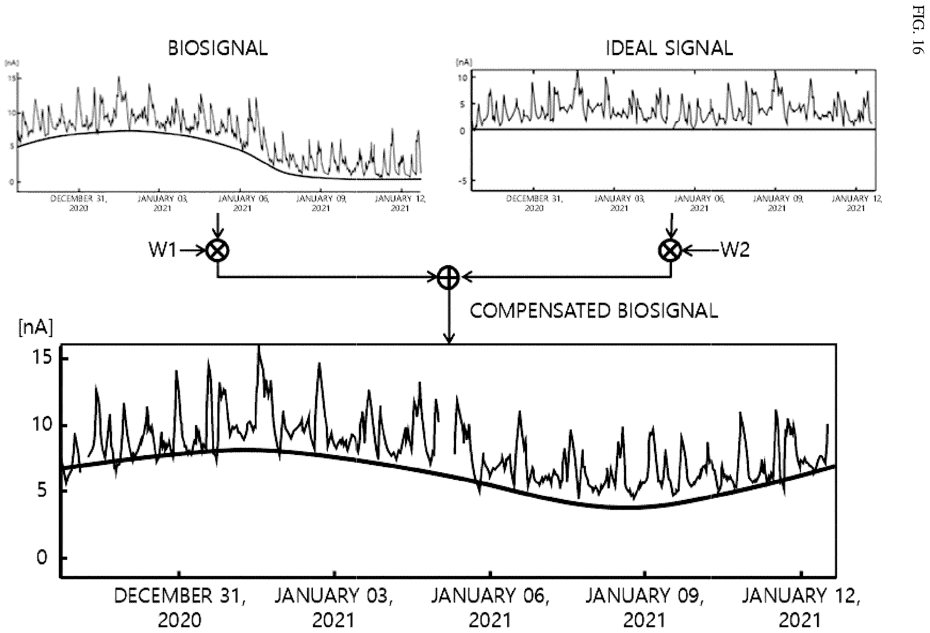

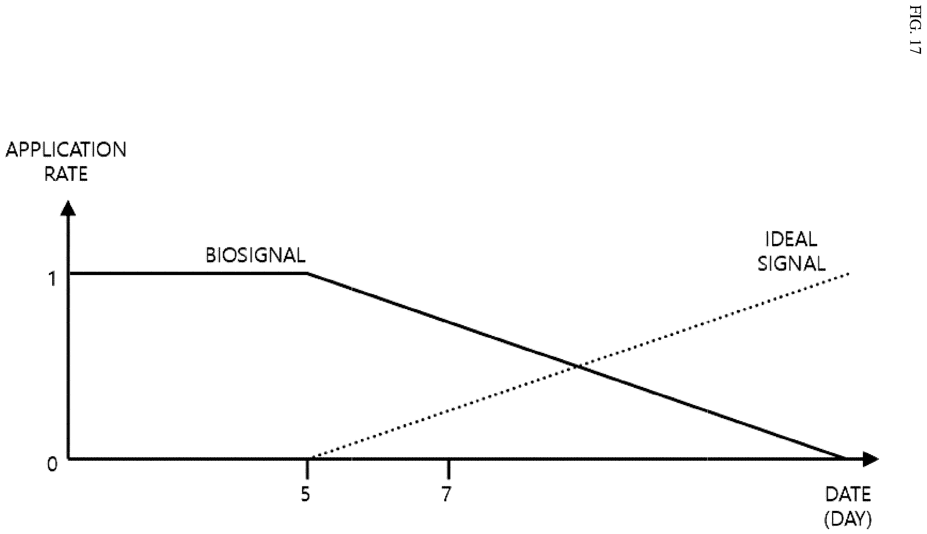

FIG. 16 is a diagram for describing a process of generating compensation data, which is second biometric information compensated from second biometric information and ideal data, according to an embodiment of the present invention. FIG. 17 is a diagram for describing a weight applied to the second biometric information and the ideal data according to an embodiment.

As described above, the compensation unit 140 may generate multiple weights, and acquire a first weight applied to the second biometric information and a second weight applied to the ideal data. The compensation unit 140 may apply the first weight to the second biometric information and the second weight to the ideal data to generate the compensation data (i.e., the second biometric information from which the syllable fragmentation phenomenon has been removed). The compensation unit 140 may acquire the glucose level from the compensation data and output the acquired glucose level to the user.

The compensation unit 140 may apply the first weight and the second weight, which change over time, to the second biometric information and the ideal data to generate the compensated second biometric information, and may add up the results to generate the compensated second biometric information. The compensation unit 140 may apply a first weight W1 to the second biometric information obtained from the sensor transmitter 10, and a second weight W2 to the ideal data processed from the second biometric information. The compensation unit 140 may add up the result of applying the first weight W1 to the second biometric information and the result of applying the second weight W2 to the ideal data. As a result, the compensation unit 140 may finally generate the compensation data.

In the compensation data, the change (curve) in the baseline may be formed more gently than that in the second biometric information. This is because the ideal data is reflected. As the weighted value applied to the ideal data is reflected, the baseline of the compensation data gradually increases in the syllable fragmentation stabilization section, and may be more gently bent than the baseline of the second biometric information.

In particular, the first weight W1 and the second weight W2 applied to both signals are not reflected uniformly over time, but may be reflected differently. The first weight W1 and the second weight W2 may have different application rates. Specifically, referring to FIG. 17, the first weight W1 and the second weight W2 may have a negative correlation. In addition, the first weight W1 and the second weight W2 may have values that vary between 0 (zero) and 1.

In the section (for example, for the first 5 days) where the syllable fragmentation does not occur, the compensation unit 140 may not perform real-time compensation on the second biometric information. Thereafter, when entering the section where the syllable fragmentation occurs (e.g., after 5 days), the compensation unit 140 may perform the real-time compensation on the second biometric information. Here, the compensation unit 140 may increase the first weight W1 and apply the first weight W1 to the second biometric information at the beginning of the occurrence of the syllable fragmentation, and may decrease the second weight W2 and apply the second weight W2 to the ideal data. Since the syllable fragmentation phenomenon of the second biometric information is not severe, even when the second biometric information is reflected a lot, the measurement result falls within the normal range. However, when the syllable fragmentation phenomenon becomes more and more severe and the compensation unit 140 enters the syllable fragmentation stabilization section, conversely, the compensation unit 140 may decrease the first weight W1 to apply the first weight W1 to the second biometric information, and increase the second weight W2 to apply the second weight W2 to the ideal data. Since the syllable fragmentation phenomenon of the second biometric information is severe, it is necessary to adjust the measurement result by reflecting the ideal data more than the second biometric information. For example, the first weight W1 may be 1, 0.9, 0.8, 0.7, and 0.6 for 5, 6, 7, 8, 9, and 10 days, and conversely, the second weight W2 may be 0, 0.1, 0.2, 0.3, and 0.4. In this way, two weights do not necessarily need to change equally. When the compensation unit 140 may use the first weight W1 and the second weight W2 that change over time and perform the compensation in real time by adapting to the occurrence of the syllable fragmentation phenomenon, the weights may change in various forms.

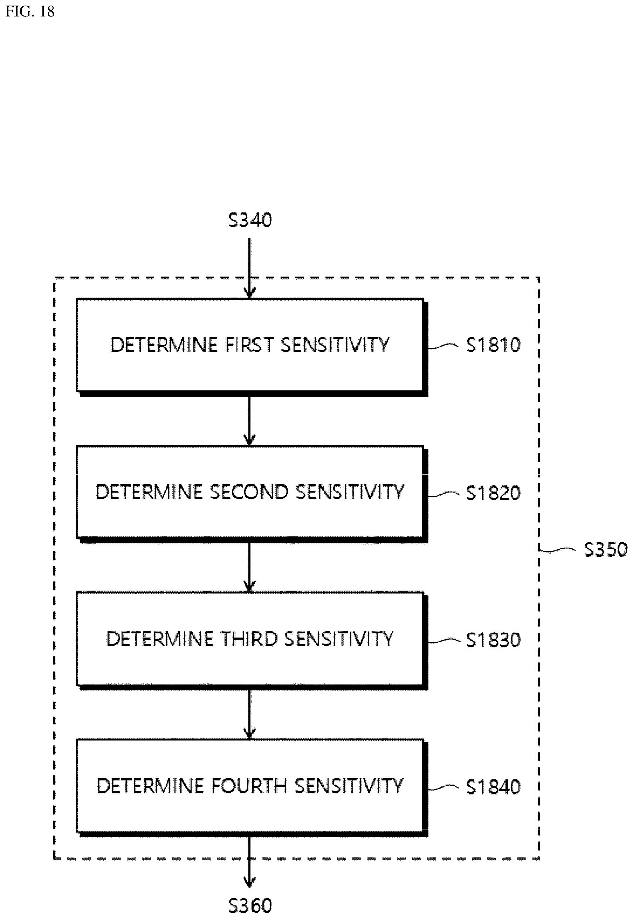

FIG. 18 is a flowchart of another embodiment illustrating in detail step S350 of FIG. 3.

Referring to FIGS. 3 and 18, the compensation unit 140 may determine the first sensitivity based on the compensation data and the set reference glucose level. The compensation unit 140 may adjust the first sensitivity to determine the final sensitivity. The compensation unit 140 may determine a first sensitivity based on the compensation data and a set reference glucose level. A specific embodiment thereof may be as follows.

Referring to FIG. 18, the compensation unit 140 may determine the first sensitivity based on the compensation data and the set reference glucose level (S1810). Specifically, the compensation unit 140 may determine the sensitivity from the compensation data in which the offset value is reflected and the reference glucose level. In this case, the compensation unit 140 may use the following Equation 1.

$$[\text{Equation 1}]$$

$$S = \frac{BGr}{C - Voff}$$

When the above Equation 1 is used to determine the first sensitivity, S may denote the first sensitivity, BGr may denote the reference glucose level, and C-Voff may denote the compensation data.

The compensation unit 140 may perform a second sensor sensitivity determination process of determining the second sensitivity based on the sensor sensitivity and the first sensitivity that are set in response to the glucose measurement sensor (S1820).

Here, the sensor sensitivity set in response to the glucose measurement sensor may be a sensitivity preset for any one sensor, or a sensitivity preset for any one group (e.g., a manufacturing lot) that is a set of the plurality of sensors. The sensitivity for a group of sensors may be understood as a specific sensitivity that represents the entire sensor of one group, rather than just one sensor. For the convenience of description, the sensor sensitivity preset for the sensor may be called 'nominal sensitivity' .

Here, the nominal sensitivity may include the ideal sensitivity expected from one sensor or a group of sensors under ideal conditions. For example, the nominal sensitivity may be understood as the sensitivity that one sensor or a group of sensors should have under ideal conditions such as a laboratory environment. Alternatively, the nominal sensitivity may be understood as a sensitivity preset at a factory when one sensor or a group of sensors is manufactured. When the terminal is used, the nominal sensitivity needs to be coded for initialization, in which the nominal sensitivity may be input to and/or stored in the terminal by being included in the coding information during the coding process.

Describing step S1820 in detail, the compensation unit 140 may determine the normal sensitivity as the second sensitivity when the first sensitivity is higher than equal to the nominal sensitivity.

On the other hand, the compensation unit 140 may determine the nominal sensitivity, which is processed through the set algorithm, as the second sensitivity when the first sensitivity is lower than the sensor sensitivity.

The compensation unit 140 may perform a third sensor sensitivity determination process of adjusting the second sensitivity based on the first sensitivity and the final sensitivity that are determined at a previous time point and determining the adjusted second sensitivity as the third sensitivity (S1830).

Specifically, the compensation unit 140 may determine the average value of the first sensitivity and the second sensitivity as the third sensitivity when the state in which the first sensitivity determined at the previous time point is higher or lower than the final sensitivity at corresponding previous time point continuously occurs. Here, the previous time point may mean a specific time point in the past before the current time point at which the sensitivity is to be calibrated. For example, the compensation unit 140 may determine that the reliability of the first sensitivity is high when the first sensitivity is at least twice higher or lower than the sensitivity at the first previous time point. The compensation unit 140 may acquire the combined sensitivity by reflecting the first sensitivity and the second sensitivity and determine the combined sensitivity as the third sensitivity. The compensation unit 140 may acquire the combined sensitivity by averaging the first sensitivity and the second sensitivity.

The compensation unit 140 may perform the third sensor sensitivity determination process of adjusting the third sensitivity according to whether the glucose level acquired based on the third sensitivity falls within the set range and determining the adjusted third sensitivity as a fourth sensitivity (S1840).

Specifically, the compensation unit 140 may acquire the glucose level related to the compensation data using the third sensitivity.

The compensation unit 140 may determine the third sensitivity as the fourth sensitivity when the glucose level falls within the preset range. Here, the preset range may be specified by a previous glucose level and a reference glucose level. The compensation unit 140 may acquire the previous glucose level from a current value at the current time point, the final sensitivity at the previous time point, and the offset value acquired at the previous time point. The compensation unit 140 may acquire the reference glucose level from the outside. The compensation unit 140 may determine whether the glucose level acquired from the current value at the current time point, the offset value at the current time point, and the third sensitivity falls within the range defined by the previous glucose level and the reference glucose level.

On the other hand, the compensation unit 140 may determine the fourth sensitivity based on the glucose level at the previous time point, the reference glucose level, and the compensation data when the glucose level does not fall within the preset range.

According to an embodiment, the compensation unit 140 may determine any one of the first sensitivity, the second sensitivity, the third sensitivity, and the fourth sensitivity as the final sensitivity.

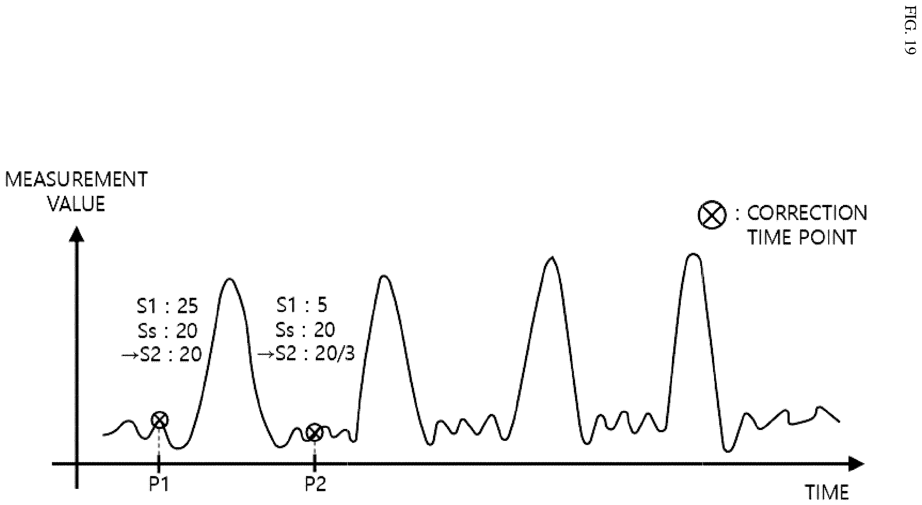

FIG. 19 is a diagram for describing the process of determining the second sensitivity according to an embodiment of the present invention.

Referring to FIG. 19, an example of determining the second sensitivity from the first sensitivity and the nominal sensitivity may be illustrated. The compensation unit 140 of the terminal may reflect the nominal sensitivity to adjust the first sensitivity, and determine the second sensitivity as the result. However, the compensation unit 140 of the terminal may reflect the nominal sensitivity differently in some cases.

First, the compensation unit 140 may compare the first sensitivity with the nominal sensitivity. When the first sensitivity is higher than the nominal sensitivity, the compensation unit 140 may determine the nominal sensitivity as the second sensitivity. For example, in the drawing, when a first sensitivity S1 is 25 at a first calibration time point P1 and a nominal sensitivity Ss, which is a sensitivity by the coding information, is 20, the compensation unit 140 may determine a second sensitivity S2 as 20. For the convenience of description, the unit will be omitted below. The compensation unit 140 may more trust the nominal sensitivity Ss to determine the nominal sensitivity Ss as the second sensitivity S2.

When the first sensitivity is lower than the nominal sensitivity, the compensation unit 140 may process the nominal sensitivity and determine the processed second sensitivity as the first sensitivity. For example, in FIG. 19, when the first sensitivity S1 is 5 at the second calibration time point P2 and the nominal sensitivity Ss, which is the sensitivity by the coding information of the sensor, is 20, the compensation unit 140 may process the nominal sensitivity Ss to acquire a value related to 1/3 of the nominal sensitivity Ss, and determine 20/3 (≒ 6.7), which is a value related to 1/3 of the nominal sensitivity Ss, as the second sensitivity S2. The compensation unit 140 may more trust the nominal sensitivity Ss to determine the processed nominal sensitivity Ss as the second sensitivity S2.

When the first sensitivity is equal to the nominal sensitivity, the compensation unit 140 may determine the first sensitivity as the second sensitivity as it is.

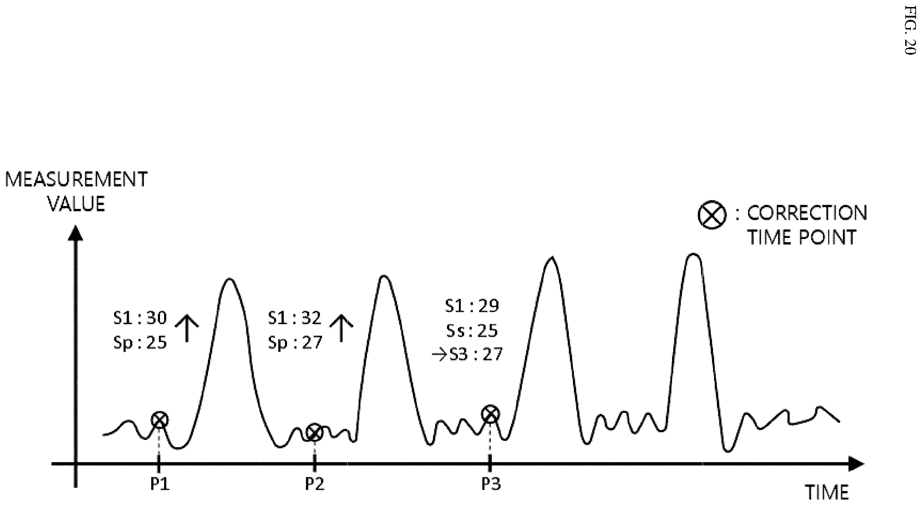

FIG. 20 is a diagram for describing the process of determining the third sensitivity according to an embodiment of the present invention.

Referring to FIG. 20, an example of adjusting the second sensitivity to determine the third sensitivity may be illustrated. For example, in FIG. 20, when the first sensitivity S1 is 30 and a previous sensitivity Sp is 25 at the first calibration time point P1 and the first sensitivity S1 is 32 and the previous sensitivity Sp is 27 at the second calibration time point P2, since the first sensitivity S1 has a higher value than the previous sensitivity Sp at two consecutive calibration time points, the compensation unit 140 may determine that the reliability of the first sensitivity S1 having 29 at the third calibration time point P3 is high. The compensation unit 140 may acquire the combined sensitivity from the first sensitivity S1 29 and the second sensitivity S2 25 at the third calibration time point P3. The compensation unit 140 may acquire 27 (= (29+25)/2), which is an average value of the first sensitivity S129 and the second sensitivity S2 25, as the combined sensitivity. The compensation unit 140 may determine the combined sensitivity 27 as the third sensitivity S3.

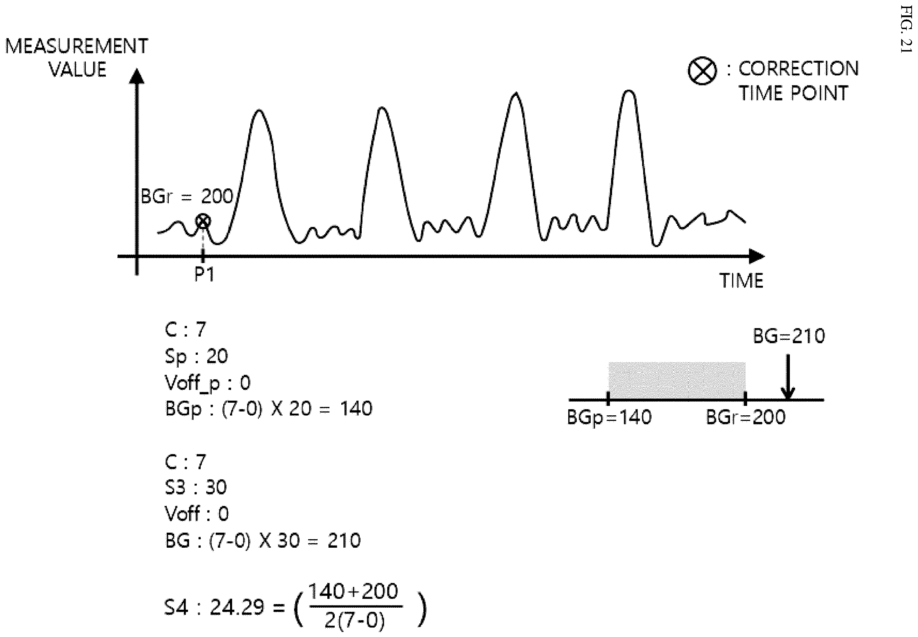

FIG. 21 is a diagram for describing the process of determining the fourth sensitivity according to an embodiment of the present invention.

Referring to FIG. 21, an example of determining the fourth sensitivity from the third sensitivity according to whether the glucose level acquired from the third sensitivity falls within a certain range may be illustrated. In an example, it may be assumed that the compensation unit 140 determines the fourth sensitivity at the first calibration time point P1. First, the compensation unit 140 may specify a set range and determine the validity of the third sensitivity.

When a current value C at the first calibration time point P1 is 7, the previous sensitivity Sp is 20, and a previous offset value Voff_p is 0, a previous glucose level BGp may be 140 (= (7-0) ×20). Here, the previous sensitivity Sp and the previous offset value Voff_p may be the sensitivity and offset value determined before the sensitivity calibration is performed at the first calibration time point P1. When the reference glucose level BGr is 200 at the first calibration time point P1, the glucose level range in which the glucose level by the third sensitivity is valid, that is, a certain range specified by the previous glucose level and the reference glucose level, may correspond to 140 to 200.

Since the current value C at the first calibration time point P1 is 7, the offset value Voff at the first calibration time point P1 is 0, and the third sensitivity S3 is 30, the compensation unit 140 may acquire the glucose level BG 210 (= (7-0) ×30) from these values. Since the glucose level BG is 210 which does not fall within a certain range of 140 to 200, the compensation unit 140 may consider the third sensitivity S3 to be invalid and acquire the adjustment sensitivity to determine the fourth sensitivity.

The compensation unit 140 may acquire the adjustment sensitivity based on the current value (at the current time point), the offset value (at the current time point), the glucose level at the previous time point, and the reference glucose level using the following Equation 2.

[Equation 2]

$$Sa = \frac{BGp + BGr}{2} \cdot \frac{1}{C - Voff}$$

According to this example, the current value C at the first calibration time point P1 is 7, the offset value Voff at the first calibration time point P1 is 0, the glucose level BGp at the previous time point is 140, and the reference glucose level BGr is 200, so the adjustment sensitivity Sa may be 24.29. The compensation unit 140 may determine the adjustment sensitivity Sa as the fourth sensitivity. The compensation unit 140 may determine 24.29 as the fourth sensitivity.

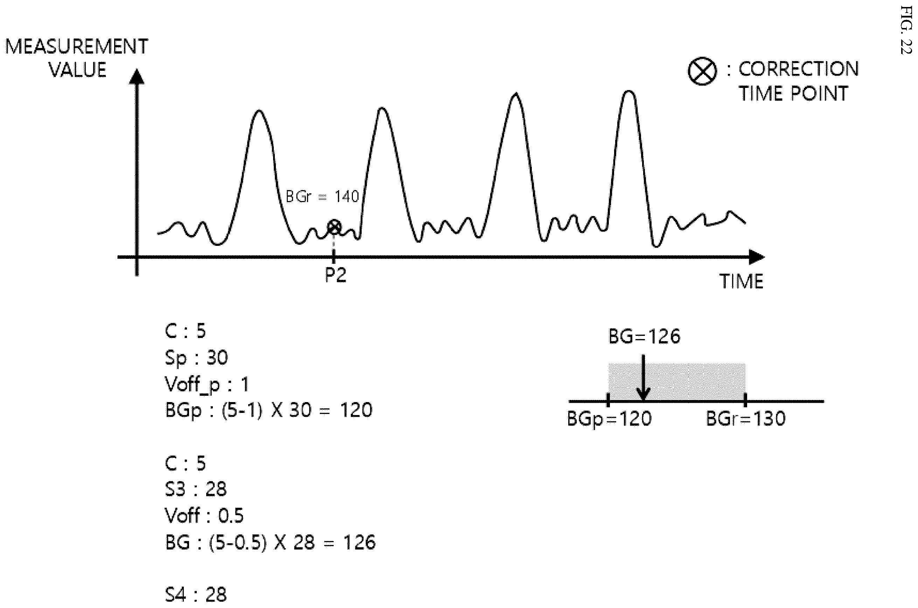

FIG. 22 is a diagram for describing another embodiment that determines the fourth sensitivity according to an embodiment of the present invention.

Referring to FIG. 22, another example of determining the fourth sensitivity from the third sensitivity according to whether the glucose level derived from the third sensitivity falls within the set range may be illustrated. In another example, it may be assumed that the compensation unit 140 determines the fourth sensitivity at the second calibration time point P2. First, the compensation unit 140 may specify a certain range and determine the validity of the third sensitivity.

When the current value C at the second calibration time point P2 is 5, the final sensitivity Sp at the previous time point is 30, and the offset value Voff_p at the previous time point is 1, the glucose level BGp at the previous time point may be 120 (= (5-1) ×30). Here, the previous sensitivity Sp and the previous offset value Voff_p may be the sensitivity and offset value determined before the sensitivity calibration is performed at the second calibration time point P2. When the reference glucose level BGr is 140 at the second calibration time point P2, the glucose level range in which the glucose level by the third sensitivity is valid, that is, a certain range specified by the previous glucose level and the reference glucose level, may correspond to 120 to 140.

Since the current value C at the second calibration time point P2 is 5, the offset value Voff at the second calibration time point P2 is 0.5, and the third sensitivity S3 is 28, the compensation unit 140 may acquire the glucose level BG 126 (= (5-0.5) ×28) from these values. Since the glucose level BG is 126, which falls within a certain range of 120 to 140, the compensation unit 140 may consider the third sensitivity S3 to be valid and determine the third sensitivity S3 as the fourth sensitivity. Therefore, the fourth sensitivity in this example may be 28.

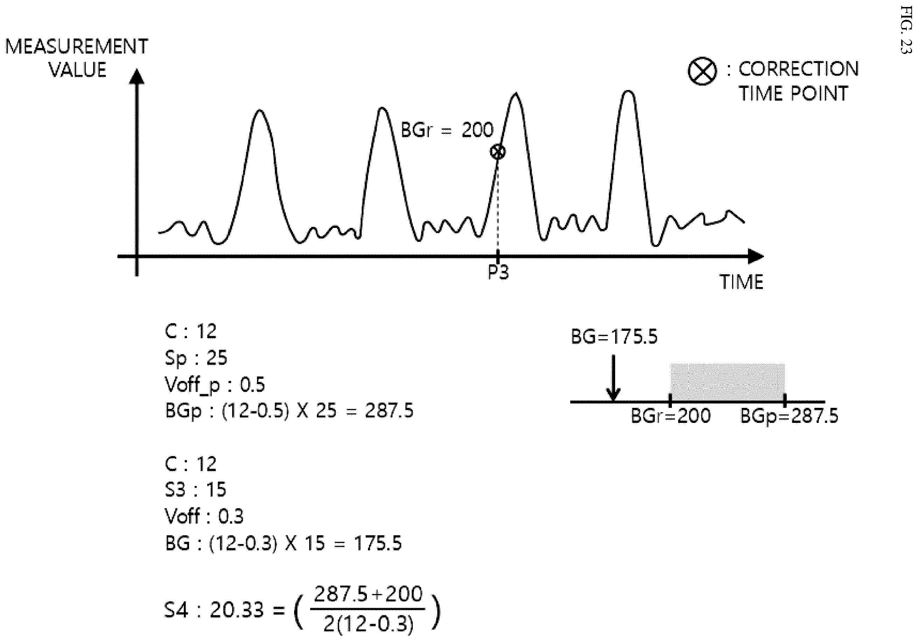

FIG. 23 is a diagram for describing another embodiment that determines the fourth sensitivity according to an embodiment of the present invention.

Referring to FIG. 23, another example of determining the fourth sensitivity from the third sensitivity according to whether the glucose level acquired from the third sensitivity falls within a certain range may be illustrated. In another example, the compensation unit 140 may be assumed to determine the fourth sensitivity at the third calibration time point P3. First, the compensation unit 140 may specify a certain range and determine the validity of the third sensitivity.

When the current value C at the third calibration time point P3 is 12, the previous sensitivity Sp is 25, and the previous offset value Voff_p is 0.5, a previous glucose level BGp may be 287.5 (= (12-0.5) ×25). Here, the previous sensitivity Sp and the previous offset value Voff_p may be the sensitivity and offset value determined before the sensitivity calibration is performed at the third calibration time point P3. When the reference glucose level BGr is 200 at the third calibration time point P3, the glucose level range in which the glucose level by the third sensitivity is valid, that is, a certain range specified by the previous glucose level and the reference glucose level, may correspond to 200 to 287.5.

Since the current value C at the third calibration time point P3 is 12, the offset value Voff at the third calibration time point P3 is 0.3, and the third sensitivity S3 is 15, the compensation unit 140 may acquire the glucose level BG 175.5 (= (12-0.3) ×15) from these values. Since the glucose level BG is 175.5 which does not fall within a certain range of 200 to 287.5, the compensation unit 140 may consider the third sensitivity S3 to be invalid and acquire the adjustment sensitivity to determine the fourth sensitivity.

The compensation unit 140 may acquire the adjustment sensitivity based on the current value (at the current time point), the offset value (at the current time point), the previous glucose level and the reference glucose level using the above Equation 3. According to this example, the current value C at the third calibration time point P3 is 12, the offset value Voff at the third calibration time point P3 is 0.3, the previous glucose level BGp is 287.5, and the reference glucose level BGr is 200, so the adjustment sensitivity Sa may be 20.33. The compensation unit 140 may determine 20.33 as the fourth sensitivity.

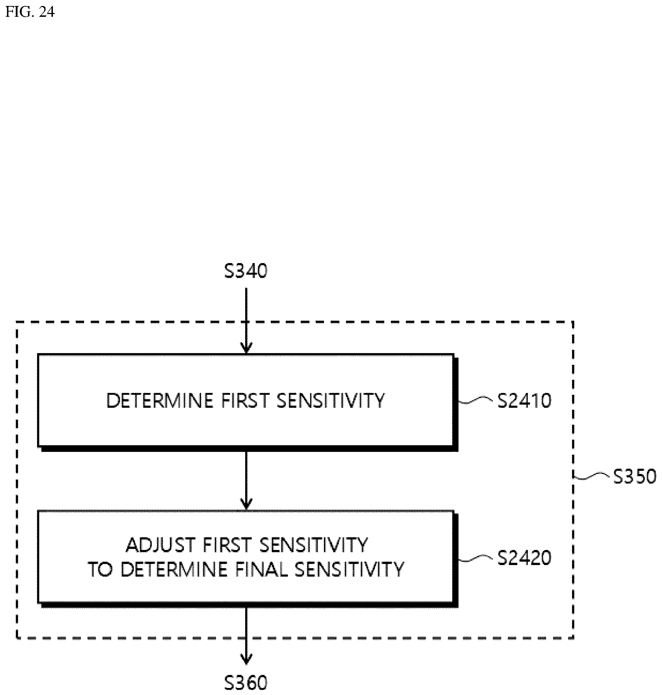

Hereinafter, other examples of the sensitivity acquisition process of acquiring the calibration algorithm will be described with reference to FIGS. 24 to 28.

FIG. 24 is a flowchart illustrating a method of acquiring sensitivity according to another embodiment.

Referring to FIG. 24, the method of acquiring sensitivity according to another embodiment illustrated in FIG. 24 may be illustrated. The compensation unit 140 of the apparatus 100 for monitoring glucose may first determine the sensitivity required to generate the glucose level from the current value, and then the glucose level acquisition unit 150 may apply the current value to the finally determined sensitivity to generate the glucose level. In addition, in another embodiment, the description of an embodiment including the same configuration as the embodiment is applied as it is. Hereinafter, the description common to one embodiment will be omitted and the difference will be mainly described.

First, the compensation unit 140 may determine the first sensitivity (S2410). The process of determining the first sensitivity has been described above through other embodiments, and therefore, a detailed description thereof will be omitted.

Next, the compensation unit 140 may perform the adjustment on the first sensitivity. The compensation unit 140 may adjust the first sensitivity to determine the final sensitivity (S2420). In order to determine the final sensitivity from the first sensitivity, the compensation unit 140 may perform the following detailed adjustment.

Specifically, the compensation unit 140 may adjust the first sensitivity according to the period that has elapsed since the sensor is inserted into the body, or adjust the first sensitivity based on an average sensing value obtained by averaging the plurality of sensing values and an average glucose level obtained by averaging the plurality of glucose levels. Here, when the first sensitivity is adjusted according to the period that has elapsed since the sensor is inserted into the body, the adjusted first sensitivity may be named 'sensitivity A'. In addition, when the first sensitivity is adjusted based on the average sensing value obtained by averaging the plurality of sensing values and the average glucose level obtained by averaging the plurality of glucose levels, the adjusted first sensitivity may be named as 'sensitivity B'.

The compensation unit 140 may determine either the sensitivity A or the sensitivity B as the adjusted first sensitivity, i.e., the final sensitivity that the compensation unit 140 obtains. Here, the compensation unit 140 may selectively apply any one of the two methods described above to adjust the first sensitivity. Alternatively, the compensation unit 140 may sequentially apply both of the two methods described above to adjust the first sensitivity. Through this step, either the sensitivity A or the sensitivity B may be determined from the first sensitivity, and either the sensitivity A or the sensitivity B may be determined as the final sensitivity for acquiring the glucose level from the current value.

For example, in the case of the selective application, the compensation unit 140 may generate the sensitivity A from the first sensitivity and determine the generated sensitivity A as the final sensitivity. Alternatively, the compensation unit 140 may generate the sensitivity B from the first sensitivity and determine the generated sensitivity B as the final sensitivity. As another example, in the case of the sequential application, the compensation unit 140 may generate the sensitivity A from the first sensitivity, generate the sensitivity B from the sensitivity A, and determine the generated sensitivity B as the final sensitivity. Alternatively, the sensitivity B may be generated from the first sensitivity of apparatus 100 for monitoring glucose, the sensitivity A may be generated from the sensitivity B, and the generated sensitivity A may be determined as the final sensitivity. Hereinafter, the steps of determining the sensitivity A and determining the sensitivity B will be described in detail.

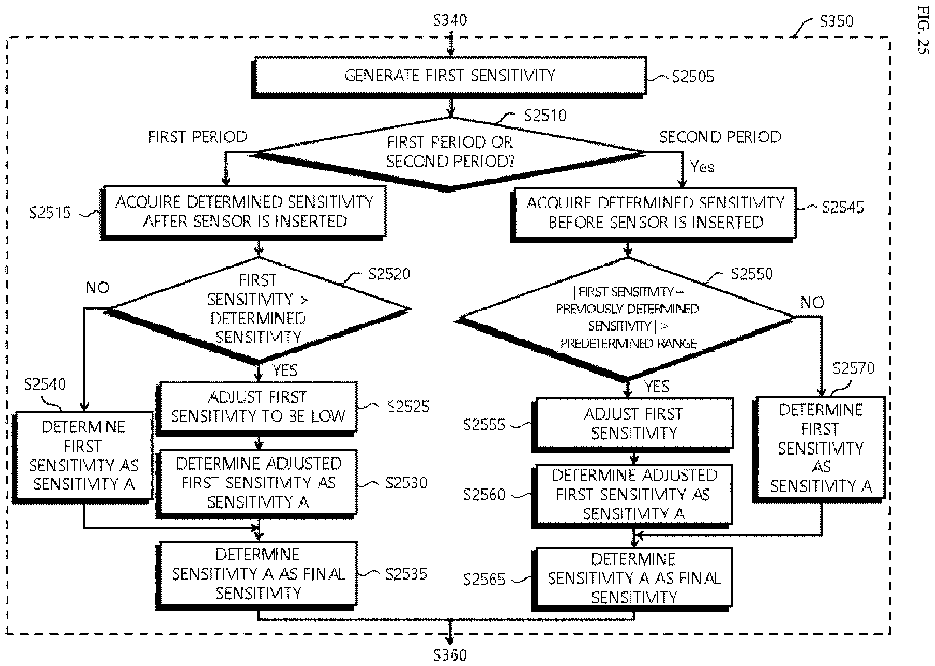

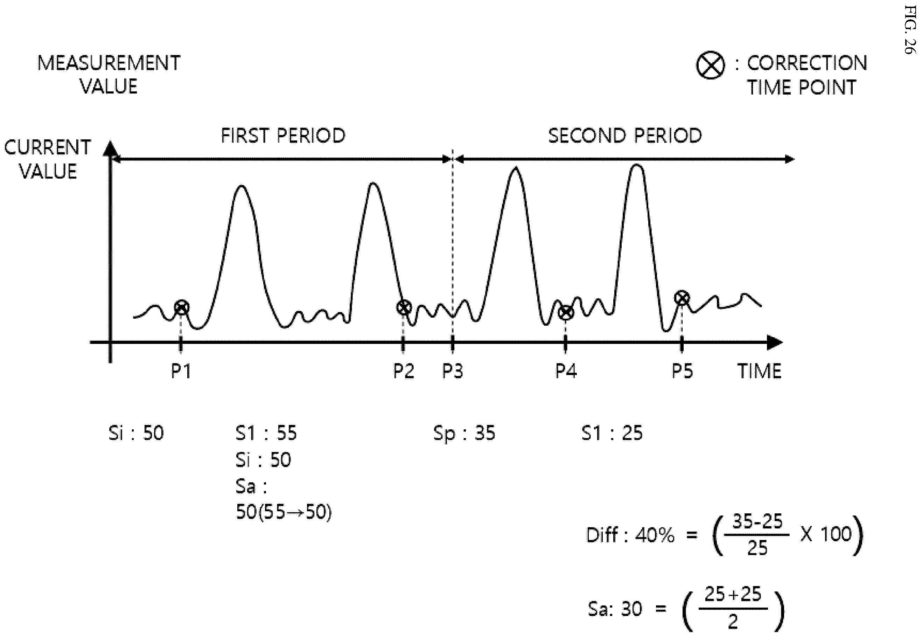

FIG. 25 is a flowchart illustrating a method of adjusting sensitivity according to the period that has elapsed since the sensor according to FIG. 24 is inserted into the body, and FIG. 26 is an exemplary diagram of adjusting sensitivity according to the period that has elapsed since the sensor according to FIG. 25 is inserted into the body.

Referring to FIG. 25, a method of adjusting sensitivity, that is, a method of determining sensitivity A according to a period that has elapsed since a sensor according to another embodiment has been inserted into a body may be illustrated.

As described above, the compensation unit 140 may generate the first sensitivity (S2505).

In addition, the compensation unit 140 may determine how much time has elapsed since the sensor has been inserted into the body (S2510). It may be determined whether the current time point at which the sensitivity is determined belongs to the first period or the second period. The first period and the second period may be divided based on whether a hydration process of a sensor occurs. Therefore, the first period may mean a period (i.e., a period during which the hydration process exists) from when the sensor is inserted into a body to when the hydration process ends, and the second period may mean a period (i.e., a period during which the hydration process does not exist) after the hydration process ends. The first period and the second period may be divided based on the end of the hydration process. The time point (i.e., the end time point of the hydration process) at which the first period and the second period are divided may be determined based on the change pattern of the current value or may be preset by the user by a certain time. The first sensitivity may be adjusted in different ways according to whether the current time point belongs to the first period or the second period.

First, when the compensation unit 140 determines that the current time point belongs to the first period, the first sensitivity may be adjusted based on the sensitivity determined after the sensor is inserted into the body. To this end, the compensation unit 140 may acquire the sensitivity determined after the sensor is inserted into the body (S2515). Preferably, the sensitivity determined after the sensor is inserted into the body may be the sensitivity determined for the first time after the sensor is inserted. In the early stages (within 2 to 3 days) after the sensor is inserted, due to the hydration process, the current value may increase and thus the sensitivity may tend to decrease. However, when the first sensitivity is higher than the sensitivity determined after the sensor is inserted, the first sensitivity is considered abnormal and the first sensitivity needs to be adjusted lower to match the sensitivity determined after the sensor is inserted. Therefore, since the sensitivity determined 'initially' after the sensor is inserted is derived during the hydration process, the need for adjustment of the first sensitivity may be made more reasonable.

The compensation unit 140 can determine whether the first sensitivity exceeds the sensitivity determined after the sensor is inserted into the body (step S2520). When the first sensitivity exceeds the sensitivity determined after the sensor is inserted into the body, the compensation unit 140 may adjust the first sensitivity to be low (step S2525). Preferably, the first sensitivity may be adjusted to be low to the sensitivity determined 'initially' after the sensor is inserted into the body. The compensation unit 140 may determine the adjusted first sensitivity as the sensitivity A (step S2530). The sensitivity A is determined as the final sensitivity (S2535), and the glucose level acquisition unit 150 may acquire the glucose level for the current value through the sensitivity A.

When the first sensitivity does not exceed the sensitivity determined after the sensor is inserted into the body, the compensation unit 140 may determine the first sensitivity as the sensitivity A without adjusting the first sensitivity (S2540). The sensitivity A is determined as the final sensitivity (S2535), and the glucose level acquisition unit 150 may acquire the glucose level for the current value through the sensitivity A.

Meanwhile, when the compensation unit 140 determines that the current time point falls within the second period, the first sensitivity may be adjusted based on the sensitivity determined before the current time point. For this purpose, the compensation unit 140 may acquire the sensitivity determined before (the current time point) (S2545). Since the second period is a period in which the hydration process ends and no longer occurs, the adjustment like the adjustment in the first period may not be required. In the second period, since the 'stabilization' section with little change in current value comes, it may be reasonable that there is little difference between the previously determined sensitivity and the first sensitivity at the current time point. Therefore, when the first sensitivity has a large difference compared to the previously determined sensitivity (at the current time point), it may be reasonable that the first sensitivity is adjusted to be close to the previously determined sensitivity.

The compensation unit 140 may determine whether the first sensitivity exceeds the previously determined sensitivity. To this end, the compensation unit 140 may determine whether the difference between the first sensitivity and the previously determined sensitivity exceeds a certain range (S2550). When the difference between the first sensitivity and the previously determined sensitivity exceeds a certain range, the compensation unit 140 may adjust the first sensitivity so that the difference falls within a certain range. To this end, the compensation unit 140 may adjust the first sensitivity by averaging the first sensitivity and the previously determined sensitivity (S2555). The compensation unit 140 may determine the average value as the adjusted first sensitivity. The compensation unit 140 may determine the adjusted first sensitivity as the sensitivity A (step S2560). The sensitivity A is determined as the final sensitivity, so the compensation unit 140 may acquire the glucose level for the current value through the sensitivity A (S2565). On the other hand, when the difference between the first sensitivity and the previously determined sensitivity does not exceed a certain range, the compensation unit 140 may determine the first sensitivity as the sensitivity A without adjusting the first sensitivity (S2570). The sensitivity A is determined as the final sensitivity (S2565), so the compensation unit 140 may acquire the glucose level for the current value through the sensitivity A.

Referring to FIG. 26, an example of adjusting sensitivity according to a period that has elapsed since a sensor according to another embodiment is inserted into the body may be illustrated. The compensation unit 140 may adjust the first sensitivity in different ways according to the insertion period of the sensor.

First, describing the method in which the first sensitivity is adjusted in a first period X1 in which the hydration process occurs after the sensor is inserted, the first calibration time point P1 may be the time point at which the sensitivity is determined 'for the first time' after the sensor is inserted. The sensitivity determined at the first calibration time point P1, that is, the sensitivity Si initially determined after the sensor is inserted, may be 50. For the convenience of description, the unit will be omitted. Thereafter, the second calibration time point P2 may be the time point at which the compensation unit 140 wants to determine the final sensitivity. At the second calibration time point P2, the compensation unit 140 may acquire the first sensitivity S1 of 55, and determine that the first sensitivity S1 exceeds a sensitivity Si initially determined after the sensor is inserted. The first sensitivity S1 may be adjusted from 55 to 50, and this adjusted first sensitivity may be the sensitivity A. Therefore, in the first period X1, the sensitivity A may be 50.

The hydration process may end at the third calibration time point P3. After the third calibration time point P3, a second period X2 may begin.

Next, describing the method of adjusting a first sensitivity in the second period X2 when the hydration process ends and the current value is stabilized, a fourth calibration time point P4 may be the time point related to the previously determined sensitivity (previous sensitivity). The compensation unit 140, which wants to determine the sensitivity at a fifth calibration time point P5, may use the sensitivity determined at the fourth calibration time point P4. The sensitivity determined at the fourth calibration time point P4, that is, the previous sensitivity Sp, may be 35. Thereafter, at the fifth calibration time point P5, the compensation unit 140 may acquire the first sensitivity S1 of 25, and determine that a difference Diff between the first sensitivity S1 and the previous sensitivity Sp is 40%, which does not fall within a certain range (e.g., certain range is 20%). The difference Diff may be represented as a ratio of the difference between the first sensitivity S1 and the previous sensitivity Sp to the first sensitivity S1, and when a numerical value is applied, the difference Diff may be 40%. The certain range may be the same concept as a preset threshold value. The first sensitivity S1 may be adjusted to fall within a certain range, in which the first sensitivity S1 may be averaged together with the previous sensitivity Sp. The compensation unit 140 may adjust the first sensitivity S1 to 30 by averaging the first sensitivity S1 25 and the previous sensitivity Sp 35. The adjusted first sensitivity may be the sensitivity A. Therefore, in the second period X2, the sensitivity A may be 30.

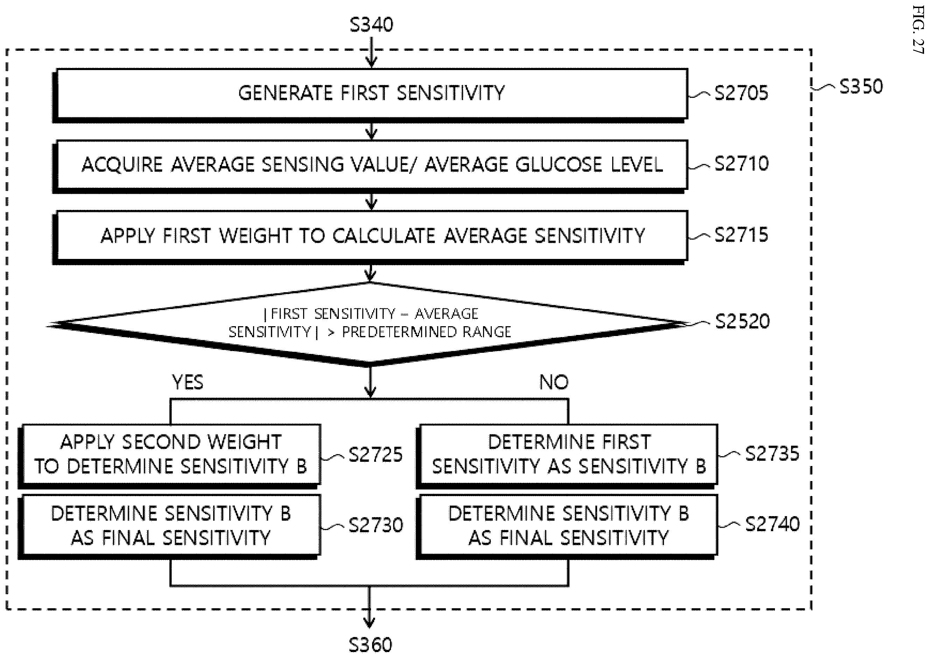

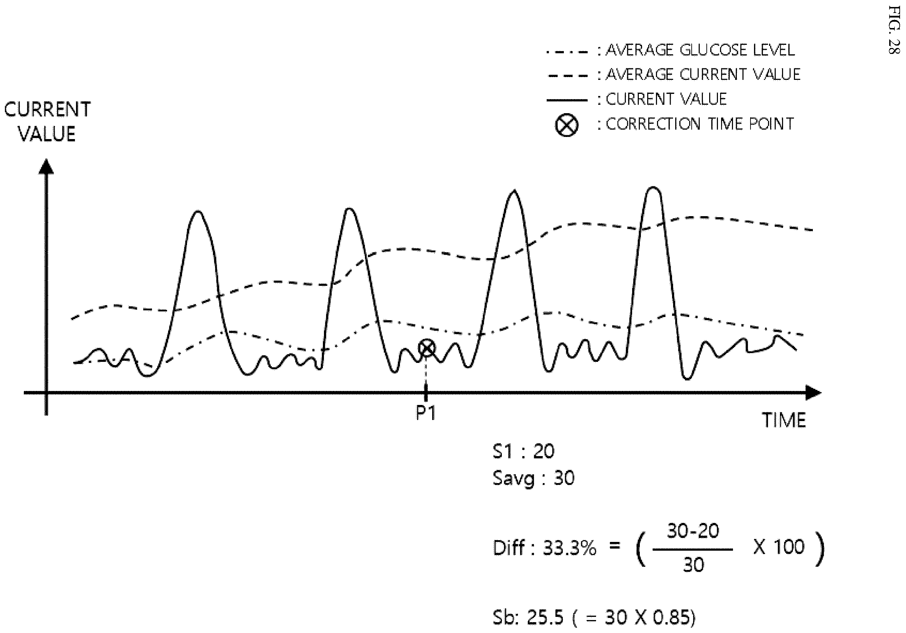

FIG. 27 is a flowchart illustrating a method of adjusting sensitivity based on the average sensing value and the average glucose level illustrated in FIG. 24, and FIG. 28 is an exemplary diagram for adjusting sensitivity based on the average sensing value and the average glucose level illustrated in FIG. 27.

Referring to FIG. 27, a method of adjusting sensitivity, that is, a method of determining sensitivity B, based on the average sensing value and the average glucose level according to another embodiment may be illustrated.

As described above, the compensation unit 140 may generate the first sensitivity (S2705). The compensation unit 140 may acquire the average sensing value and the average glucose level (S2710). The average sensing value may mean the average value of the sensing value measured by the sensor up to the current time point at which the sensitivity is calibrated. A starting time point of the average may be the time point at which the sensor is inserted or any one time point before the current time point preset by a user. The sensing values from the starting time point to the current time point may be averaged. In addition, the average glucose level may mean the average value of the glucose level acquired from the CGMS up to the current time point at which the sensitivity is calibrated. As with the average sensing value, the starting time point of the average may be determined, and the glucose level from the time point to the current time point may be averaged.

Next, the compensation unit 140 may apply the first weight to the first sensitivity, the average sensing value, and the average glucose level to acquire the average sensitivity (S2715). The first weight may be assigned to each of the first sensitivity, the average sensing value, and the average glucose level, and each of the first weights may change over time. For example, the compensation unit 140 may apply a 1a-th weight to the first sensitivity, a 1b-th weight to the average sensing value, and a 1c-th weight to the average glucose level. As the 1b-th and 1c-th weights are adjusted, the ratio reflected in the first sensitivity may increase or decrease.

The compensation unit 140 may acquire the difference between the first sensitivity and the average sensitivity and determine whether the difference exceeds a certain range (S2720). When the difference exceeds a certain range, the compensation unit 140 may determine the sensitivity B from the average sensitivity by using the average sensitivity instead of the first sensitivity (S2725). When the first sensitivity is excessively spaced apart from the average sensitivity, the average sensitivity is used. The compensation unit 140 may apply the second weight to the average sensitivity to determine the average sensitivity as the sensitivity B. The sensitivity B is determined as the final sensitivity (S2730), and the glucose level acquisition unit 150 may acquire the glucose level for the current value through the sensitivity B.

When the difference does not exceed a certain range, the compensation unit 140 may determine the first sensitivity as the sensitivity B without using the average sensitivity (S2735). The sensitivity B is determined as the final sensitivity (S2740), so the compensation unit 140 may acquire the glucose level for the current value through the sensitivity B.

Referring to FIG. 28, an example of adjusting the sensitivity based on the average sensing value and the average glucose level according to another embodiment may be illustrated. In this drawing, the current value related to the sensing value, and the average current value and the average glucose level related to the average sensing value may be illustrated together.

The first calibration time point P1 is the current time point, which may be the time point at which the compensation unit 140 attempts to acquire the final sensitivity in order to acquire the glucose level. At the first calibration time point P1, the compensation unit 140 may acquire the first sensitivity S1 of 20. The compensation unit 140 may apply the first weight to the average sensing value, the average glucose level, and the first sensitivity S1 to acquire the average sensitivity Savg of 30.

The compensation unit 140 may determine the sensitivity B based on the average sensitivity Savg. Specifically, the compensation unit 140 may acquire the difference Diff between the first sensitivity S1 and the average sensitivity Savg. The difference Diff may be represented as the ratio of the difference between the first sensitivity S1 and the average sensitivity Savg to the average sensitivity Savg, and when the value is applied, the difference Diff may be 33.3%. It may be determined that the difference Diff does not fall within a certain range (e.g., a certain range is 20%). The certain range may be the same concept as a preset threshold value. The average sensitivity Savg may be used to determine the sensitivity B, and the second weight may be applied to the average sensitivity Savg. The second weight may be set to 0.85, and the sensitivity B may be 25.5. Here, the second weight may change to increase over time. As a result, the ratio of the average sensitivity Savg reflected in the sensitivity B may increase. This may be because the average sensitivity (Savg) reflecting the past trend may be more accurate as time passes.

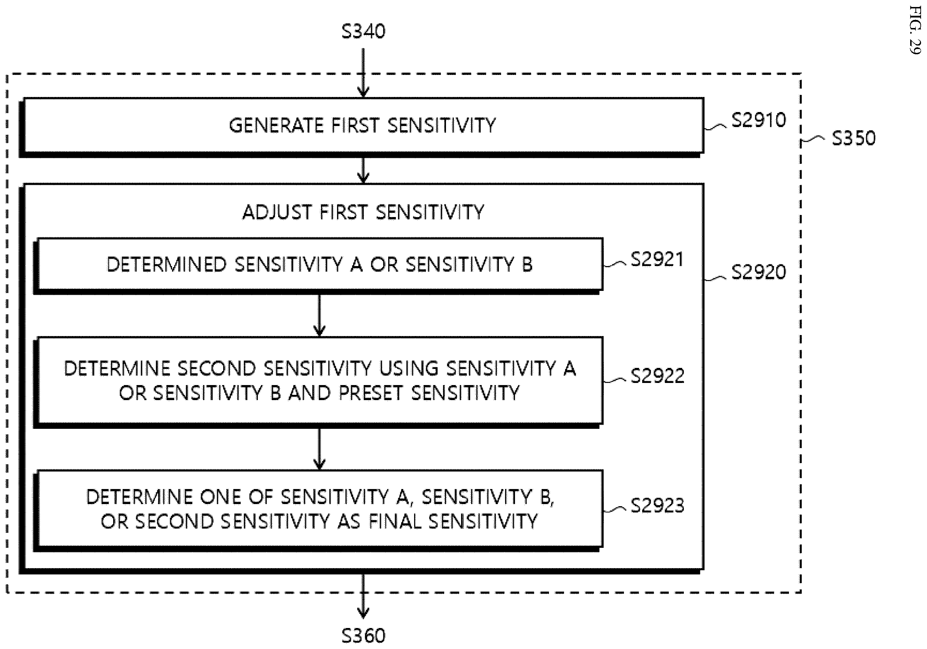

FIG. 29 is a flowchart illustrating a first example of a method of calibrating sensitivity illustrated in FIG. 24 according to another embodiment.

Referring to FIG. 29, first, the compensation unit 140 may determine the first sensitivity as described above (S2910).

Next, the adjustment for the first sensitivity may be performed (S2920). The compensation unit 140 may adjust the first sensitivity to finally determine the sensitivity. In order to finally determine the sensitivity from the first sensitivity, the compensation unit 140 may adjust the first sensitivity in detail as follows.

The compensation unit 140 may adjust the first sensitivity based on the period that has elapsed since the sensor was inserted into the body to determine the sensitivity A, or may adjust the first sensitivity based on the average sensing value obtained by averaging the plurality of sensing values and the average glucose level obtained by averaging the plurality of glucose levels to determine the sensitivity B (S2921).

In addition, the compensation unit 140 may determine the second sensitivity from the sensitivity A or the sensitivity B and the sensitivity preset according to the sensor (S2922). Determining the second sensitivity based on the sensitivity preset according to the sensor may be the same as described above in an embodiment.

The compensation unit 140 may determine any one of the sensitivity A, the sensitivity B, and the second sensitivity as the adjusted first sensitivity, that is, the final sensitivity that the compensation unit 140 obtains (S2923). Here, the compensation unit 140 may acquire the sensitivity A and the sensitivity B in S2921, and may also acquire the second sensitivity based on any one of the sensitivity A and the sensitivity B in S2922. The compensation unit 140 may determine any one of the sensitivity A, the sensitivity B, and the second sensitivity as the final sensitivity for acquiring the glucose level from the current value. The compensation unit 140 may adjust the first sensitivity through S2921 to S2923 to determine the sensitivity.

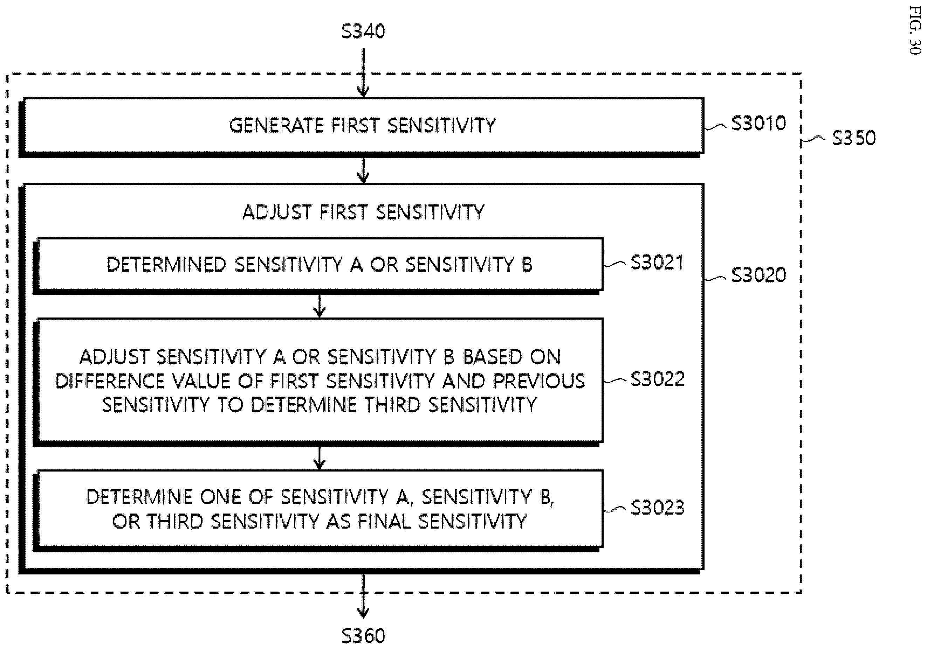

FIG. 30 is a flowchart illustrating a second example of the method of calibrating sensitivity illustrated in FIG. 24 according to another embodiment.

Referring to FIG. 30, first, the compensation unit 140 may determine the first sensitivity as described above (S3010).

Next, the adjustment for the first sensitivity may be performed. The compensation unit 140 may adjust the first sensitivity to finally determine the sensitivity (S3020). In order to finally determine the sensitivity from the first sensitivity, the compensation unit 140 may adjust the first sensitivity in detail as follows.

The compensation unit 140 may adjust the first sensitivity based on the period that has elapsed since the sensor was inserted into the body to determine the sensitivity A, or may adjust the first sensitivity based on the average sensing value obtained by averaging the plurality of sensing values and the average glucose level obtained by averaging the plurality of glucose levels to determine the sensitivity B (S3021).

In addition, the compensation unit 140 may adjust the sensitivity A or the sensitivity B according to the reliability based on the difference between the first sensitivity and the previously determined sensitivity to determine the third sensitivity (S3022). Determining the third sensitivity based on the reliability based on the difference between the first sensitivity and the previously determined sensitivity may be the same as described above in an embodiment.

The compensation unit 140 may determine any one of the sensitivity A, the sensitivity B, and the third sensitivity as the adjusted first sensitivity, that is, the final sensitivity that the compensation unit 140 obtains (S3023). Here, the compensation unit 140 may acquire the sensitivity A and the sensitivity B in S3021, and may also determine the third sensitivity based on any one of the sensitivity A and the sensitivity B in S3022. The compensation unit 140 may determine any one of the sensitivity A, the sensitivity B, and the third sensitivity as the final sensitivity for acquiring the glucose level from the current value. The compensation unit 140 may adjust the first sensitivity through S3021 to S3023 to determine the sensitivity.

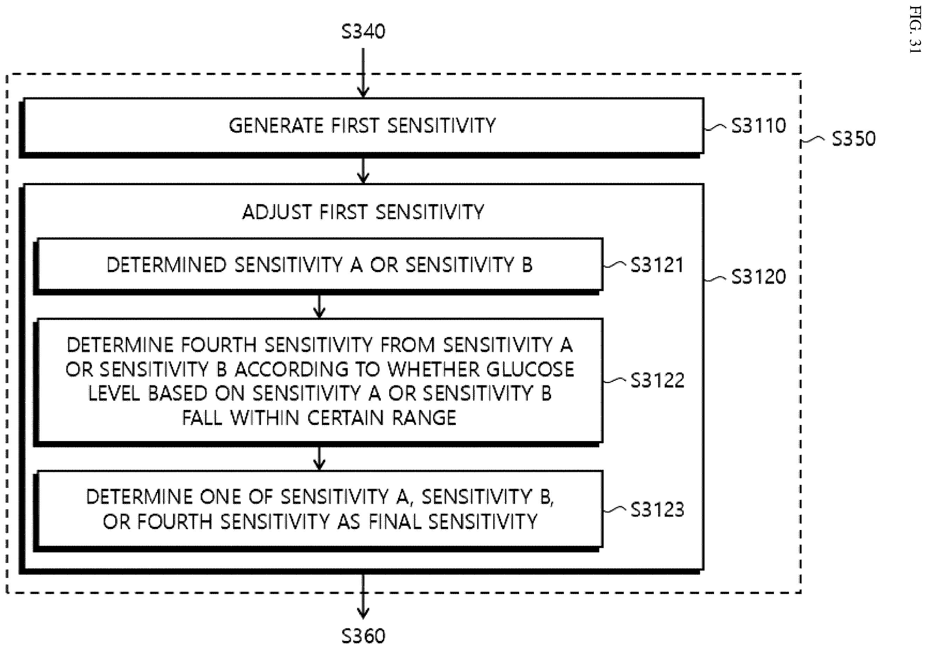

FIG. 31 is a flowchart illustrating a third example of the method of calibrating sensitivity illustrated in FIG. 24 according to another embodiment.

Referring to FIG. 31, first, the compensation unit 140 may determine the first sensitivity as described above (S3110).

Next, the adjustment for the first sensitivity may be performed. The compensation unit 140 may adjust the first sensitivity to finally determine the sensitivity (S3120). In order to finally determine the sensitivity from the first sensitivity, the compensation unit 140 may adjust the first sensitivity in detail as follows.

The compensation unit 140 may adjust the first sensitivity based on the period that has elapsed since the sensor was inserted into the body to determine the sensitivity A, or may adjust the first sensitivity based on the average sensing value obtained by averaging the plurality of sensing values and the average glucose level obtained by averaging the plurality of glucose levels to determine the sensitivity B (S3121).

In addition, the compensation unit 140 may determine the fourth sensitivity from the sensitivity A or the sensitivity B according to whether the offset value and the glucose level acquired from the sensitivity A or the sensitivity B fall within a certain range (S3122). Determining the fourth sensitivity may be the same as described above in an embodiment.

The compensation unit 140 may determine any one of the sensitivity A, the sensitivity B, and the fourth sensitivity as the adjusted first sensitivity, that is, the final sensitivity that the compensation unit 140 obtains (S3123). Here, the compensation unit 140 may acquire the sensitivity A and the sensitivity B in S3121, and may also determine the fourth sensitivity based on any one of the sensitivity A and the sensitivity B in S3122. The compensation unit 140 may determine any one of the sensitivity A, the sensitivity B, and the fourth sensitivity as the final sensitivity for acquiring the glucose level from the current value. The compensation unit 140 may adjust the first sensitivity through S3121 to S3123 to determine the sensitivity.

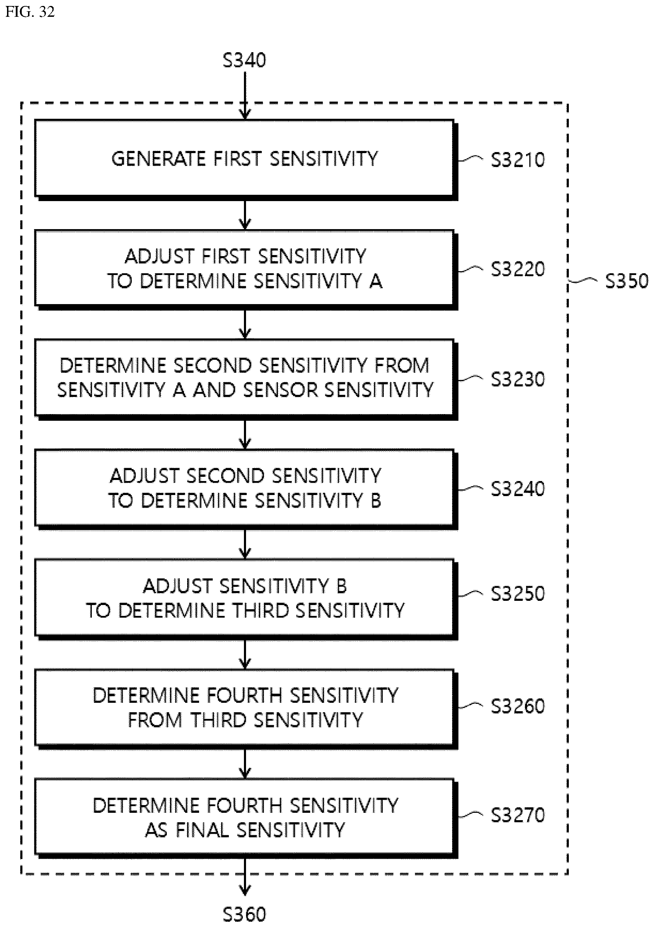

FIG. 32 is a flowchart illustrating the method of calibrating sensitivity according to another embodiment.

Referring to FIG. 32, a method for calibrating sensitivity in the apparatus 100 for monitoring glucose constituting a CGMS according to another embodiment may be illustrated. In the method illustrated in FIG. 24, each step of determining the sensitivity A, the sensitivity B and the second to fourth sensitivities may be applied selectively or in parallel, whereas in the method illustrated in FIG. 32, each step may be applied serially so as to have one order.

First, the compensation unit 140 may determine the first sensitivity as described above (S3110).

In addition, the compensation unit 140 may acquire the first sensitivity, and adjust the first sensitivity according to the period that has elapsed since the sensor is inserted to determine the sensitivity A (S3220).

In addition, the compensation unit 140 may acquire the sensitivity A and determine the second sensitivity from the sensitivity A and the sensor sensitivity (e.g., nominal sensitivity) (S3230).

In addition, the compensation unit 140 may adjust the second sensitivity based on the average sensing value obtained by averaging the plurality of sensing values and the average glucose level obtained by averaging the plurality of glucose levels to determine the sensitivity B (S3240).

In addition, the compensation unit 140 may acquire the sensitivity B, and adjust the sensitivity B according to the reliability based on the difference between the first sensitivity and the previously determined sensitivity to determine the third sensitivity (S3250).

In addition, the compensation unit 140 may acquire the third sensitivity, and determine the fourth sensitivity from the third sensitivity according to whether the glucose level acquired from the offset value and the third sensitivity falls within a certain range (S3260).

The compensation unit 140 may determine the fourth sensitivity as the final sensitivity (S3270).

The term '~ unit' used in the present embodiment refers to software or a hardware component such as a field programmable gate array (FPGA) or an application specific integrated circuit (ASIC), and '~ unit' play certain roles. However, '~ unit' is not limited to the software or the hardware. The "~unit" may be configured to be stored in a storage medium that can be addressed or may be configured to regenerate one or more processors. Accordingly, as an example, the "~unit" refers to components such as software components, object-oriented software components, class components, and task components, processes, functions, attributes, procedures, subroutines, segments of program code, drivers, firmware, microcode, circuits, data, databases, data structures, tables, arrays and variables. Components and functions provided within "~unit" may be combined into a smaller number of components and "~unit" or may be further separated into additional components and "~unit." Furthermore, components and '~ units' may be implemented to reproduce one or more central processing units (CPUs) in a device or a security multimedia card.

Although the above description has been made based on the embodiments, this is only an example and does not limit the present invention. Those skilled in the art to which the present invention pertains may understand that several modifications and applications that are not described in the present specification may be made without departing from the spirit of the present invention. For example, each component described in detail in the embodiment may be modified and implemented. In addition, differences associated with these modifications and applications are to be interpreted as falling within the scope of the present invention as defined by the following claims.

**Claims**

1. An apparatus for monitoring glucose, comprising:

   an input unit that obtains first biometric information including a plurality of data points measuring glucose-related information of a subject;
   a noise removal unit that removes noise information included in the first biometric information;
   a preprocessing unit that preprocesses the first biometric information from which the noise information is removed to generate second biometric information having a lower sampling rate than the first biometric information;
   a compensation unit that generates compensation data based on the second biometric information and generates a calibration algorithm based on the compensation data; and
   a glucose level acquisition unit that acquires a glucose level related to the second biometric information by reflecting the calibration algorithm and a set time delay.

2. The apparatus of claim 1, wherein the noise removal unit performs a first noise removal process of removing a data point determined as noise information among first data points based on a plurality of data points included in a set time interval, and
   performs a second noise removal process of adjusting a value of the first data point based on a plurality of data points included in a set time interval to remove set noise information.

3. The apparatus of claim 2, wherein the noise removal unit performs the first noise removal process of:

   determining whether any one of the data points is an outlier using a data point other than any one of the plurality of data points included in the set time interval; and
   removing any one of the data points based on being determined that any one of the data points is the outlier.

4. The apparatus of claim 3, wherein the noise removal unit performs the first noise removal process of:

   acquiring a reference value including at least one of an average slope, a slope change value, an average value, and a standard deviation using the data point other than any one of the plurality of data points; and
   comparing the reference value with the one data point to determine whether the one data point is the outlier.

5. The apparatus of claim 2, wherein the noise removal unit performs the second noise removal process of:

   acquiring a weight for any one of the data points using a data point other than any one of the plurality of data points; and
   applying the weight to any one of data points to remove a noise component included in any one of data points.

6. The apparatus of claim 1, wherein the preprocessing unit performs a first preprocessing process of preprocessing the first biometric information from which the noise information is removed based on the first biometric information from which the noise information is removed and a first time interval.

7. The apparatus of claim 6, wherein the preprocessing unit performs the first preprocessing process of:

   dividing a plurality of data points included in the first biometric information from which the noise information is removed at the first time interval; removing a first data point related to a set upper value and a second data point related to a set lower value among a plurality of data points at each first time interval; and

acquiring an average value of the plurality of data points from which the first data point and the second data point are removed at each first time interval as the first preprocessing data.

8. The apparatus of claim 7, wherein the preprocessing unit performs a second preprocessing process of preprocessing the first preprocessing data based on the first preprocessing data and a second time interval having a value greater than the first time interval.

9. The apparatus of claim 8, wherein the preprocessing unit performs the second preprocessing process of:

dividing a plurality of average values included in the first preprocessing data at the second time interval;

removing a first average value related to a set upper value and a second average value related to a set lower value among the plurality of average values at each second time interval;

acquiring an average value for the plurality of average values from which the first average value and the second average value are removed at each second time interval as second preprocessing data; and

determining the second preprocessing data as the second biometric information.

10. The apparatus of claim 8, wherein the compensation unit determines any one of values of pre-stored table data as an offset value based on the second biometric information, and

applies the offset value to the second biometric information to generate the compensation data.

11. The apparatus of claim 1, wherein the compensation unit acquires an average value of data points for a set period included in the second biometric information,

applies the average value to the second biometric information to generate ideal data having a set baseline,

generates a first weight and a second weight, and

adds up a value obtained by applying the first weight to the second biometric information and a value obtained by applying the second weight to the ideal data to generate the compensation data.

12. The apparatus of claim 1, wherein the compensation unit performs at least one of:

a first sensor sensitivity determination process of determining a first sensitivity based on the compensation data and a set reference glucose level;

a second sensor sensitivity determination process of determining a second sensitivity based on a sensor sensitivity set in response to a glucose measurement sensor and the first sensitivity;

a third sensor sensitivity determination process of adjusting the second sensitivity based on a first previous time point sensitivity and a first previous time point final sensitivity that are determined at a previous time point and determining the adjusted second sensitivity as the third sensitivity; and

a fourth sensor sensitivity determination process of adjusting the third sensitivity according to whether a glucose level acquired based on the third sensitivity falls within a set range and determining the adjusted third sensitivity as a fourth sensitivity, and

determines any one of the first sensitivity, the second sensitivity, the third sensitivity, and the fourth sensitivity as the final sensitivity.

13. The apparatus of claim 13, wherein the compensation unit performs the second sensor sensitivity determination process of: determining the sensor sensitivity as the second sensitivity based on that the first sensitivity is higher than or equal to the sensor sensitivity; and determining the processed sensor sensitivity, which is obtained by processing the sensor sensitivity through a set algorithm, as the second sensitivity based on that the first sensitivity is lower than the sensor sensitivity, and/or

the compensation unit performs the third sensor sensitivity determination process of determining an average value of the first sensitivity and the second sensitivity as the third sensitivity based on that a state in which the first sensitivity determined at a previous time point is higher or lower than the final sensitivity of the corresponding previous time point occurs continuously, and/or

the compensation unit performs the fourth sensor sensitivity determination process of: acquiring a glucose level related to the compensation data using the third sensitivity; determining the third sensitivity as the fourth sensitivity when the glucose level falls within a preset range; and determining the fourth sensitivity based on a glucose level at the previous time point, a reference glucose level, and the compensation data when the glucose level does not fall within a preset range.

14. The apparatus of claim 1, wherein the glucose level acquisition unit compensates for the time delay in the second biometric information to generate time-compensated biometric information, and
applies the time-compensated biometric information to the calibration algorithm to acquire the glucose level.

15. A method of monitoring glucose using an apparatus for monitoring glucose, comprising:

obtaining, by the apparatus for monitoring glucose, first biometric information including a plurality of data points measuring glucose-related information of a subject;
removing, by the apparatus for monitoring glucose, noise information included in the first biometric information;
preprocessing, by the apparatus for monitoring glucose, the first biometric information from which the noise information is removed to generate second biometric information having a lower sampling rate than the first biometric information;
generating, by the apparatus for monitoring glucose, compensation data based on the second biometric information; and
generating a calibration algorithm based on the compensation data; and
acquiring, by the apparatus for monitoring glucose, a glucose level related to the second biometric information by reflecting the calibration algorithm and a set time delay.

FIG. 1

FIG. 2

INPUT UNIT ~ 110

NOISE REMOVAL UNIT ~ 120

PREPROCESSING UNIT ~ 130

COMPENSATION UNIT ~ 140

GLUCOSE LEVEL
ACQUISITION UNIT ~ 150

~ 100

FIG. 3

START

INPUT FIRST BIOMETRIC INFORMATION — S310

REMOVE NOISE INCLUDED IN FIRST BIOMETRIC INFORMATION — S320

GENERATE SECOND BIOMETRIC INFORMATION BASED ON FIRST BIOMETRIC INFORMATION — S330

GENERATE COMPENSATION DATA BASED ON SECOND BIOMETRIC INFORMATION — S340

GENERATE CALIBRATION ALGORITHM BASED ON COMPENSATION DATA — S350

ACQUIRE GLUCOSE LEVEL BASED ON CALIBRATION ALGORITHM AND SECOND BIOMETRIC INFORMATION — S360

END

FIG. 4

FIG. 5

OT

B6

B5

B3

B4

B1

B2

EP 4 574 039 A1

30

FIG. 6

FIG. 7

S320

S330

**FIRST PREPROCESSING PROCESS** — S710

DIVIDE PLURALITY OF DATA POINTS AT FIRST TIME INTERVAL — S711

REMOVE UPPER VALUE AND LOWER VALUE FOR EACH DIVIDED RANGE — S712

GENERATE, FOR EACH DIVIDED RANGE, AVERAGE VALUE OF REMAINING DATA POINT AS FIRST PREPROCESSING DATA — S713

**SECOND PREPROCESSING PROCESS** — S720

DIVIDE FIRST PREPROCESSING DATA AT SECOND TIME INTERVAL — S721

REMOVE UPPER VALUE AND LOWER VALUE FOR EACH DIVIDED RANGE — S722

GENERATE, FOR EACH DIVIDED RANGE, AVERAGE VALUE OF REMAINING DATA POINT AS SECOND PREPROCESSING DATA — S723

DETERMINE SECOND PREPROCESSING DATA AS SECOND BIOMETRIC INFORMATION — S724

S340

FIG. 8

FIG. 9

FIG. 10

FIG. 11

FIG. 12

FIG. 13

EP 4 574 039 A1

S330

DETERMINE OFFSET VALUE IN TABLE DATA
BASED ON SECOND BIOMETRIC INFORMATION — S1310

— S340

APPLY OFFSET VALUE TO SECOND BIOMETRIC INFORMATION
TO GENERATE COMPENSATION DATA — S1320

S350

FIG. 14

S340

S330 →

**S1410** — ACQUIRE AVERAGE VALUE FROM THE SECOND BIOMETRIC INFORMATION FOR SET PERIOD

**S1420** — GENERATE IDEAL DATA BASED ON AVERAGE VALUE

**S1430** — ACQUIRE FIRST WEIGHT AND SECOND WEIGHT

**S1440** — GENERATE COMPENSATION DATA BASED ON FIRST WEIGHT, SECOND WEIGHT, SECOND BIOMETRIC INFORMATION, AND IDEAL DATA

→ S350

FIG. 15

FIG. 16

FIG. 17

FIG. 18

FIG. 19

FIG. 20

FIG. 21

⊗ : CORRECTION TIME POINT

MEASUREMENT VALUE

TIME

BGr = 200

P1

BG=210

BGp=140

BGr=200

C : 7
Sp : 20
Voff_p : 0
BGp : (7-0) X 20 = 140

C : 7
S3 : 30
Voff : 0
BG : (7-0) X 30 = 210

$$S4 : 24.29 = \left( \frac{140+200}{2(7-0)} \right)$$

FIG. 22

MEASUREMENT
VALUE

$\otimes$ : CORRECTION
TIME POINT

BGr = 140

P2

TIME

C : 5
Sp : 30
Voff_p : 1
BGp : (5-1) X 30 = 120

C : 5
S3 : 28
Voff : 0.5
BG : (5-0.5) X 28 = 126

S4 : 28

BG=126

BGp=120    BGr=130

EP 4 574 039 A1

FIG. 23

EP 4 574 039 A1

MEASUREMENT
VALUE

$\otimes$ : CORRECTION
TIME POINT

BGr = 200

P3

TIME

C : 12
Sp : 25
Voff_p : 0.5
BGp : (12-0.5) X 25 = 287.5

C : 12
S3 : 15
Voff : 0.3
BG : (12-0.3) X 15 = 175.5

$$S4 : 20.33 = \left( \frac{287.5+200}{2(12-0.3)} \right)$$

BG=175.5

BGr=200    BGp=287.5

FIG. 24

S340

DETERMINE FIRST SENSITIVITY — S2410

ADJUST FIRST SENSITIVITY
TO DETERMINE FINAL SENSITIVITY — S2420

S350

S360

FIG. 25

S340 → S350

GENERATE FIRST SENSITIVITY — S2505

S2510 — FIRST PERIOD OR SECOND PERIOD?

FIRST PERIOD

SECOND PERIOD — Yes

S2515 — ACQUIRE DETERMINED SENSITIVITY AFTER SENSOR IS INSERTED

ACQUIRE DETERMINED SENSITIVITY BEFORE SENSOR IS INSERTED — S2545

S2520 — FIRST SENSITIVITY > DETERMINED SENSITIVITY

NO

YES

S2550 — | FIRST SENSITIVITY – PREVIOUSLY DETERMINED SENSITIVITY | > PREDETERMINED RANGE

NO

S2570

S2540 — DETERMINE FIRST SENSITIVITY AS SENSITIVITY A

ADJUST FIRST SENSITIVITY TO BE LOW — S2525

DETERMINE ADJUSTED FIRST SENSITIVITY AS SENSITIVITY A — S2530

S2555 — ADJUST FIRST SENSITIVITY

DETERMINE FIRST SENSITIVITY AS SENSITIVITY A

S2560 — DETERMINE ADJUSTED FIRST SENSITIVITY AS SENSITIVITY A

DETERMINE SENSITIVITY A AS FINAL SENSITIVITY — S2535

S2565 — DETERMINE SENSITIVITY A AS FINAL SENSITIVITY

S360

50

FIG. 26

FIG. 27

EP 4 574 039 A1

S340

$\downarrow$

GENERATE FIRST SENSITIVITY — S2705     — S350

$\downarrow$

ACQUIRE AVERAGE SENSING VALUE/ AVERAGE GLUCOSE LEVEL — S2710

$\downarrow$

APPLY FIRST WEIGHT TO CALCULATE AVERAGE SENSITIVITY — S2715

$\downarrow$

| FIRST SENSITIVITY – AVERAGE SENSITIVITY | > PREDETERMINED RANGE — S2520

YES                                    NO

| APPLY SECOND WEIGHT TO DETERMINE SENSITIVITY B — S2725 | DETERMINE FIRST SENSITIVITY AS SENSITIVITY B — S2735 |

| DETERMINE SENSITIVITY B AS FINAL SENSITIVITY — S2730 | DETERMINE SENSITIVITY B AS FINAL SENSITIVITY — S2740 |

S360

FIG. 28

EP 4 574 039 A1

· — · — : AVERAGE GLUCOSE LEVEL

— — — : AVERAGE CURRENT VALUE

——— : CURRENT VALUE

⊗ : CORRECTION TIME POINT

CURRENT
VALUE

TIME

P1

S1 : 20
Savg : 30

$$\text{Diff} : 33.3\% = \left( \frac{30-20}{30} \times 100 \right)$$

Sb: 25.5 ( = 30 X 0.85)

FIG. 29

EP 4 574 039 A1

S340

GENERATE FIRST SENSITIVITY — S2910

ADJUST FIRST SENSITIVITY

DETERMINED SENSITIVITY A OR SENSITIVITY B — S2921

DETERMINE SECOND SENSITIVITY USING SENSITIVITY A OR SENSITIVITY B AND PRESET SENSITIVITY — S2922

DETERMINE ONE OF SENSITIVITY A, SENSITIVITY B, OR SECOND SENSITIVITY AS FINAL SENSITIVITY — S2923

— S2920

— S350

S360

FIG. 30

FIG. 31

```
S340 ──→

┌─────────────────────────────────────────────────────── S350
╎
╎  ┌─────────────────────────────────┐ S3110
╎  │  GENERATE FIRST SENSITIVITY     │
╎  └─────────────────────────────────┘
╎              │
╎              ↓
╎  ┌─────────────────────────────────────────────────── S3120
╎  │  ADJUST FIRST SENSITIVITY
╎  │
╎  │  ┌──────────────────────────────────────┐ S3121
╎  │  │  DETERMINED SENSITIVITY A OR          │
╎  │  │  SENSITIVITY B                        │
╎  │  └──────────────────────────────────────┘
╎  │              │
╎  │              ↓
╎  │  ┌──────────────────────────────────────┐ S3122
╎  │  │  DETERMINE FOURTH SENSITIVITY FROM    │
╎  │  │  SENSITIVITY A OR SENSITIVITY B       │
╎  │  │  ACCORDING TO WHETHER GLUCOSE LEVEL   │
╎  │  │  BASED ON SENSITIVITY A OR SENSITIVITY │
╎  │  │  B FALL WITHIN CERTAIN RANGE          │
╎  │  └──────────────────────────────────────┘
╎  │              │
╎  │              ↓
╎  │  ┌──────────────────────────────────────┐ S3123
╎  │  │  DETERMINE ONE OF SENSITIVITY A,      │
╎  │  │  SENSITIVITY B, OR FOURTH SENSITIVITY │
╎  │  │  AS FINAL SENSITIVITY                 │
╎  │  └──────────────────────────────────────┘
╎  └───────────────────────────────────────────────────
╎              │
└──────────────┼────────────────────────────────────────

               ↓
            S360
```

FIG. 32

S340

GENERATE FIRST SENSITIVITY — S3210

ADJUST FIRST SENSITIVITY
TO DETERMINE SENSITIVITY A — S3220

DETERMINE SECOND SENSITIVITY FROM
SENSITIVITY A AND SENSOR SENSITIVITY — S3230

ADJUST SECOND SENSITIVITY
TO DETERMINE SENSITIVITY B — S3240

ADJUST SENSITIVITY B
TO DETERMINE THIRD SENSITIVITY — S3250

DETERMINE FOURTH SENSITIVITY
FROM THIRD SENSITIVITY — S3260

DETERMINE FOURTH SENSITIVITY
AS FINAL SENSITIVITY — S3270

S350

S360

Europäisches Patentamt

European Patent Office

Office européen des brevets

# EUROPEAN SEARCH REPORT

**Application Number**

EP 24 22 1988

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/330895 A1 (LEE DAVID [KR] ET AL) 20 October 2022 (2022-10-20) | 1-5,10, 14,15 | INV. A61B5/1495 |
| Y | * paragraphs [0011], [0048], [0058] - [0135]; figures 1,5,9,10 * | 6-9,12, 13 | |
| X | US 2018/008201 A1 (HAYTER GARY ALAN [US] ET AL) 11 January 2018 (2018-01-11) * paragraphs [0045] - [0115] * | 1,10, 12-15 | |
| X | US 2023/210412 A1 (COBELLI CLAUDIO [IT] ET AL) 6 July 2023 (2023-07-06) * paragraphs [0012] - [0037]; figures 3,5 * | 1,10,14, 15 | |
| Y | US 2023/039204 A1 (LEE DAVID [KR] ET AL) 9 February 2023 (2023-02-09) | 6-9,12, 13 | |
| A | * paragraphs [0058] - [0090]; figures 7-9 * | 10 | |
| A | WO 2023/229137 A1 (I SENS INC [KR]) 30 November 2023 (2023-11-30) * the whole document * & EP 4 534 009 A1 (I SENS INC [KR]) 9 April 2025 (2025-04-09) * paragraphs [0013] - [0022]; figures 1,6-8 * | 1,15 | **TECHNICAL FIELDS SEARCHED (IPC)** A61B |
| A | US 2022/095964 A1 (NISHIDA JEFFREY [US] ET AL) 31 March 2022 (2022-03-31) * claim 20 * | 1,15 | |
| A | WO 00/49941 A1 (MINIMED INC [US]) 31 August 2000 (2000-08-31) * claim 23 * | 1,15 | |
| A,P | KR 2024 0027348 A (I SENS INC [KR]) 4 March 2024 (2024-03-04) * figures 4-12 * | 11 | |

The present search report has been drawn up for all claims

| Place of search | Date of completion of the search | Examiner |
|---|---|---|
| Berlin | 8 May 2025 | Kronberger, Raphael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 22 1988

This annex lists the patent family members relating to the patent documents cited in the above-mentioned European search report.
The members are as contained in the European Patent Office EDP file on
The European Patent Office is in no way liable for these particulars which are merely given for the purpose of information.

08-05-2025

| Patent document cited in search report | | Publication date | Patent family member(s) | | Publication date |
|---|---|---|---|---|---|
| US 2022330895 | A1 | 20-10-2022 | AU 2020324823 | A1 | 17-02-2022 |
| | | | EP 4011279 | A1 | 15-06-2022 |
| | | | JP 7350976 | B2 | 26-09-2023 |
| | | | JP 2022541810 | A | 27-09-2022 |
| | | | KR 20210018628 | A | 18-02-2021 |
| | | | NZ 784491 | A | 26-04-2024 |
| | | | US 2022330895 | A1 | 20-10-2022 |
| | | | WO 2021025260 | A1 | 11-02-2021 |
| US 2018008201 | A1 | 11-01-2018 | US 2009198118 | A1 | 06-08-2009 |
| | | | US 2013281807 | A1 | 24-10-2013 |
| | | | US 2016220189 | A1 | 04-08-2016 |
| | | | US 2018008201 | A1 | 11-01-2018 |
| US 2023210412 | A1 | 06-07-2023 | EP 2986214 | A1 | 24-02-2016 |
| | | | EP 3662833 | A1 | 10-06-2020 |
| | | | US 2016073964 | A1 | 17-03-2016 |
| | | | US 2019223807 | A1 | 25-07-2019 |
| | | | US 2023210412 | A1 | 06-07-2023 |
| | | | WO 2014128638 | A1 | 28-08-2014 |
| US 2023039204 | A1 | 09-02-2023 | AU 2021235831 | A1 | 22-09-2022 |
| | | | CN 115175616 | A | 11-10-2022 |
| | | | EP 4098194 | A1 | 07-12-2022 |
| | | | JP 2023517017 | A | 21-04-2023 |
| | | | KR 20210115574 | A | 27-09-2021 |
| | | | US 2023039204 | A1 | 09-02-2023 |
| | | | WO 2021182871 | A1 | 16-09-2021 |
| WO 2023229137 | A1 | 30-11-2023 | AU 2023277965 | A1 | 09-01-2025 |
| | | | CN 119255751 | A | 03-01-2025 |
| | | | EP 4534009 | A1 | 09-04-2025 |
| | | | KR 20230165066 | A | 05-12-2023 |
| | | | WO 2023229137 | A1 | 30-11-2023 |
| US 2022095964 | A1 | 31-03-2022 | US 2019175079 | A1 | 13-06-2019 |
| | | | US 2022095964 | A1 | 31-03-2022 |
| WO 0049941 | A1 | 31-08-2000 | AT E363228 | T1 | 15-06-2007 |
| | | | AU 3243500 | A | 14-09-2000 |
| | | | CA 2353486 | A1 | 31-08-2000 |
| | | | DE 60035025 | T2 | 24-01-2008 |
| | | | EP 1154718 | A1 | 21-11-2001 |
| | | | JP 2002541883 | A | 10-12-2002 |
| | | | US 6424847 | B1 | 23-07-2002 |
| | | | WO 0049941 | A1 | 31-08-2000 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 24 22 1988

08-05-2025

| Patent document cited in search report | Publication date | Patent family member(s) | Publication date |
|---|---|---|---|
| KR 20240027348 A | 04-03-2024 | NONE | |

page 2 of 2